# EUROPEAN PATENT APPLICATION

(11) **EP 3 420 901 A1**
(43) Date of publication of application: **02.01.2019**
(21) Application number: 16891635.1
(22) Date of filing: 29.11.2016
(51) Int. Cl.: A61B 5/1172, C09J 7/02, C09J 175/06, C09J 175/08

(54) **SHEET FOR IDENTIFICATION**

(30) Priority: 23.02.2016 JP 2016032235
(71) Applicant: Nitto Denko Corporation, Ibaraki-shi, Osaka 567-8680 (JP)
(72) Inventor: NISHIO, Akinori, Ibaraki-shi Osaka 567-8680 (JP); IMOTO, Takashi, Ibaraki-shi Osaka 567-8680 (JP)
(74) Representative: Müller-Boré & Partner Patentanwälte PartG mbB
(86) International application number: PCT/JP2016/085306
(87) International publication number: WO 2017/145479

(57) **Abstract**

An identification sheet (X1) according to the present invention includes a pressure-sensitive adhesive sheet (20) including a pressure-sensitive adhesive layer (22). The pressure-sensitive adhesive layer (22) contains a polyester polyurethane as a principal component. The polyester polyurethane is typically a polyester-polyether polyurethane. The identification pressure-sensitive sheet (X1) is suitable for resisting thermal deformation in the pressure-sensitive adhesive layer (22).

## Description

### Technical Field

The present invention relates to sheets for identification (identification sheets) which are usable for collection and holding of materials to be identified (target materials).

### Background Art

Target materials to be identified, such as fingerprints and hairs, may be collected typically in a crime scene. For the collection, so-called identification sheets may be used. Such an identification sheet typically includes a mount sheet (backing sheet) and a transparent pressure-sensitive adhesive sheet, where the pressure-sensitive adhesive sheet has an adhesive face and is laminated onto the mount sheet. The mount sheet has a preprinted box in a surface on opposite side to the transparent pressure-sensitive adhesive sheet, where various information relating to the collection of a target material will be recorded in the box.

Using the identification sheet as above, a target material is collected typically by the following procedure. Initially, the transparent pressure-sensitive adhesive sheet is removed from the mount sheet to expose the adhesive face, and the target material is attached to the exposed adhesive face of the pressure-sensitive adhesive sheet. Of fingerprints left typically at a crime scene, latent fingerprints are invisible to the naked eye. Collection of such a latent fingerprint as the target material may be performed typically by the following procedure. Initially, a predetermined surface or region where the presence of the latent fingerprint is estimated is subjected to a treatment using a fine powder such as a fine aluminum powder. When the latent fingerprint is present in the region, the treatment allows the fine powder to be attached to the latent fingerprint to thereby visualize the pattern shape (including ridges) of the fingerprint. The transparent pressure-sensitive adhesive sheet is removed from the mount sheet to expose an adhesive face, and the exposed adhesive face is once pressed to, and then removed from, the region. Thus, the visualized fingerprint is transferred onto the adhesive face of the transparent pressure-sensitive adhesive sheet. The transparent pressure-sensitive adhesive sheet to which the target material, such as a fingerprint, is attached typically in the above manner is laminated again onto the original mount sheet. In the lamination, the adhesive face side, of the transparent pressure-sensitive adhesive sheet, bearing the target material is attached onto the mount sheet. To the box in the mount sheet, data relating to the collection of the target material, such as date, place, collected material name, and reference number, are recorded, and a person such as a collector signs and seals the mount sheet on its preprinted box. The information such as the data may be used to specify the target material, which is collected and held by the identification sheet, and will be information necessary for surely providing the admissibility of evidence when the target material is employed as evidence typically for a criminal case.

Identification sheets as above are described typically in Patent Literature (PTL) 1 to 4 as follows.

### Citation List

### Patent Literature

PTL 1: Japanese Unexamined Patent Application Publication (JP-A) No. 2001-40299
PTL 2: JP-A No. 2003-89776
PTL 3: JP-A No. 2007-181525
PTL 4: JP-A No. 2007-181526

### Summary of Invention

### Technical Problem

An identification sheet, when designed in some ways, may undergo a step in which local temperature rise occurs in a pressure-sensitive adhesive layer in a process for producing the identification sheet. Such local temperature rise may cause thermal deformation of the pressure-sensitive adhesive layer. The present invention has been made under these circumstances and has an object to provide an identification sheet including a pressure-sensitive adhesive layer that resists or less undergoes thermal deformation. Solution to Problem

The present invention provides, in one aspect, an identification sheet. The identification sheet includes a pressure-sensitive adhesive sheet including a pressure-sensitive adhesive layer. The pressure-sensitive adhesive layer contains a polyester polyurethane as a principal component.

Such a polyester polyurethane is a polymer containing urethane bonds and ester bonds in a molecular chain. The urethane bonds in the polyester polyurethane are hydrogen-bonding functional groups each containing both a hydrogen donor group and a hydrogen acceptor group. The polyester polyurethane, which contains that kind of hydrogen-bonding functional groups, can efficiently form hydrogen bonds between its molecular chains to cohere, and has relatively high cohesive energy. This tends to allow the polyester polyurethane to actually have a high glass transition temperature and/or a high softening temperature, and, consequently, high heat resistance. The configuration, where the polyester polyurethane as above is contained as a principal component (base polymer) in the pressure-sensitive adhesive layer, tends to allow the pressure-sensitive adhesive layer in the identification sheet according to the aspect to actually have high heat resistance. The pressure-sensitive adhesive layer, with increasing heat resistance thereof, less undergoes or more resists thermal deformation.

The polyester polyurethane also contains ester bonds which are hydrogen acceptor groups, in addition to the urethane bonds as described above. In the polyester polyurethane, the ester bonds less cause molecular chains to cohere with each other, as compared with the urethane bonds. The regulation of the content or proportion of ester bonds in the polyester polyurethane can be used as one of approaches to control the cohesiveness of the polyester polyurethane. The polyester polyurethane, which contains the ester bonds as above in addition to the urethane bonds, is readily controllable on cohesive force between molecular chains within such a range as to offer sufficiently high heat resistance in the pressure-sensitive adhesive layer for use in the identification sheet. Control of the cohesiveness of the pressure-sensitive adhesive layer contributes to allowing the pressure-sensitive adhesive layer to actually have good adhesive strength and to less leave adhesive residue on an adherend after the pressure-sensitive adhesive sheet or the pressure-sensitive adhesive layer is removed from the adherend. The identification sheet tends to allow the pressure-sensitive adhesive layer to have high heat resistance while controlling the cohesiveness of the pressure-sensitive adhesive layer.

Material monomers to form the polyester polyurethane are selectable from among a wide variety of actually easily available high-purity monomers. With increasing purities of material monomers, a polymer resulting from polymerization of the material monomers tends to have higher transparency. Accordingly, the polyester polyurethane, which can be derived from material monomers selectable from among a wide variety of easily available high-purity material monomers, tends to actually have high transparency. The configuration, where the polyester polyurethane as above is contained as a principal component in the pressure-sensitive adhesive layer, tends to allow the pressure-sensitive adhesive layer in the identification sheet to actually have high transparency. Assume that the identification sheet is produced by a process through a step in which laser beams for processing typically pass through the pressure-sensitive adhesive layer. In this case, the pressure-sensitive adhesive layer, with increasing transparency thereof, less absorbs the laser beams, and, accordingly, less undergoes thermal deformation, which will be caused by laser beam absorption.

As described above, the identification sheet includes the pressure-sensitive adhesive layer that is suitable for resisting or less undergoing thermal deformation. In addition, the configuration, where the polyester polyurethane, which tends to actually offer high glass transition temperature and/or high softening temperature, is contained as a principal component in the pressure-sensitive adhesive layer, tends to allow the pressure-sensitive adhesive layer in the identification sheet to actually have high thermal stability and/or low fluidity. The high thermal stability and/or low fluidity of the pressure-sensitive adhesive layer in the identification sheet is advantageous for long-term, stable storage of a target material held in the identification sheet.

The polyester polyurethane in the pressure-sensitive adhesive layer preferably includes units derived from a multifunctional isocyanate, units derived from a polycarboxylic acid, and units derived from a first polyhydric alcohol. This configuration can appropriately give the polyester polyurethane with the technical effects.

The polyester polyurethane in the pressure-sensitive adhesive layer is preferably a polyester-polyether polyurethane. Such a polyester-polyether polyurethane contains a polyether skeleton, in addition to urethane bonds and ester bonds as described above. Ether bonds contained in the polyether skeleton have low optical absorptivity and are more flexible or movable as compared with carbon-carbon-carbon bonds. The polyester-polyether polyurethane, which contains such a polyether skeleton in addition to urethane bonds and ester bonds, tends to actually have high transparency and good flexibility while controlling the cohesive force between molecular chains within such a range as to offer sufficiently high heat resistance in the pressure-sensitive adhesive layer for use in the identification sheet. Material monomers to form polyester polyurethanes, to which the polyester-polyether polyurethane belongs, are selectable from among a wide variety of actually easily available high-purity monomers. The polyester-polyether polyurethane, which belongs to polyester polyurethanes obtainable from among a wide variety of easily available high-purity material monomers, tends to actually have high transparency. The configuration, where the polyester-polyether polyurethane as above is contained as a principal component in the pressure-sensitive adhesive layer, tends to allow the pressure-sensitive adhesive layer not only to actually have high heat resistance, but also to actually have high transparency and good flexibility, while controlling the cohesiveness of the pressure-sensitive adhesive layer in the identification sheet. The pressure-sensitive adhesive layer, with increasing heat resistance thereof, more resists or less undergoes thermal deformation, as described above. The pressure-sensitive adhesive layer, with increasing transparency thereof, more resists or less undergoes thermal deformation which will be caused by laser beam absorption, as described above. With increasing flexibility of the pressure-sensitive adhesive layer in the identification sheet, the adhesive face or the pressure-sensitive adhesive layer more readily conforms typically to, and deforms with, the surface asperities of a target material when the adhesive face of the pressure-sensitive adhesive layer is pressed onto the target material, and the surface asperities of the target material is more readily transferred onto the pressure-sensitive adhesive layer. Balancing between flexibility and thermal stability-low fluidity of the pressure-sensitive adhesive layer in the identification sheet can less cause so-called stringiness of the pressure-sensitive adhesive between an adherend and the adhesive face upon removal of the pressure-sensitive adhesive layer from the adherend, while allowing the pressure-sensitive adhesive layer or the adhesive face to actually have such tackiness as to adhere to the target material with good conformity to the asperities.

The polyester-polyether polyurethane, when contained in the pressure-sensitive adhesive layer, preferably includes units derived from a multifunctional isocyanate, units derived from a polycarboxylic acid, units derived from a first polyhydric alcohol, and units derived from a second polyhydric alcohol containing an ether bond. This configuration can appropriately give the polyester-polyether polyurethane with the technical effects.

In the polyester polyurethane in the pressure-sensitive adhesive layer, a ratio may be preferably 0.5 to 1.5, more preferably 0.7 to 1.4, furthermore preferably 0.7 to 1.3, and still more preferably 0.8 to 1.2, where the ratio is the ratio of the total number of hydroxy groups assigned to polyhydric alcohol-derived units (namely, the total number of hydroxy groups of a polyhydric alcohol to constitute the polyhydric alcohol-derived units) to the sum of the total number of isocyanate groups assigned to multifunctional isocyanate-derived units (namely, the total number of isocyanate groups of a multifunctional isocyanate to constitute the multifunctional isocyanate-derived units) and the total number of carboxy groups assigned to polycarboxylic acid-derived units (namely, the total number of carboxy groups of a polycarboxylic acid to constitute the polycarboxylic acid-derived units). With the ratio approaching 1, the polyester polyurethane tends to have a higher molecular weight and, due to such a high molecular weight, tends to have higher cohesiveness between molecules of the polyester polyurethane. With the ratio decreasing less than 1, isocyanate groups and carboxy groups tend to be present in a total number larger than the number of hydroxy groups in entire molecular ends of the polyester polyurethane, and, accordingly, the cohesiveness between molecules of the polyester polyurethane tends to be higher, where the cohesiveness is due to the presence of isocyanate groups and carboxy groups, both of which are highly polar groups which relatively highly polarize. With the ratio increasing greater than 1, hydroxy groups tend to be present in a number larger than the total number of isocyanate groups and carboxy groups in entire molecular ends of the polyester polyurethane, where the hydroxy groups have lower optical absorptivity as compared with the isocyanate groups and the carboxy groups. In this case, the polyester polyurethane therefore tends to have higher transparency. In the polyester polyurethane in the pressure-sensitive adhesive layer, the ratio of the total number of hydroxy groups of the polyhydric alcohol to the sum of the total number of isocyanate groups of the multifunctional isocyanate and the total number of carboxy groups of the polycarboxylic acid is preferably 0.5 to 1.5, more preferably 0.7 to 1.4, furthermore preferably 0.7 to 1.3, and still more preferably 0.8 to 1.2. These are preferred from the viewpoint of balance between the cohesiveness and transparency in the polyester polyurethane and, consequently, from the viewpoint of balance between the cohesiveness and transparency of the pressure-sensitive adhesive layer containing the polyester polyurethane.

In the polyester polyurethane in the pressure-sensitive adhesive layer, a ratio is preferably 0.5 to 1.0, where the ratio is the ratio of the total number of isocyanate groups assigned to multifunctional isocyanate-derived units (namely, the total number of isocyanate groups of a multifunctional isocyanate to constitute the multifunctional isocyanate-derived units) to the total number of carboxy groups assigned to polycarboxylic acid-derived units (namely, the total number of carboxy groups of a polycarboxylic acid to constitute the polycarboxylic acid-derived units). This configuration is advantageous for allowing the pressure-sensitive adhesive layer in the identification sheet to actually have both high transparency and good cohesiveness.

In the polyester-polyether polyurethane in the pressure-sensitive adhesive layer, a ratio is preferably 1.5 to 9.0, where the ratio is the ratio of the total number of hydroxy groups assigned to first polyhydric alcohol-derived units (namely, the total number of hydroxy groups of a first polyhydric alcohol to constitute the first polyhydric alcohol-derived units) to the total number of hydroxy groups assigned to second polyhydric alcohol-derived units (namely, the total number of hydroxy groups of a second polyhydric alcohol to constitute second polyhydric alcohol-derived units). This configuration is advantageous for allowing the pressure-sensitive adhesive layer in the identification sheet to surely have high heat resistance, high thermal stability, low fluidity, and high transparency and to still have both good cohesiveness and good flexibility.

In the polyester-polyether polyurethane in the pressure-sensitive adhesive layer, the second polyhydric alcohol to constitute second polyhydric alcohol-derived units is a multimer that is a polymer derived from a single type of monomer, and, in the polyester-polyether polyurethane, a ratio is preferably 0.1 to 0.4, where the ratio is the ratio of the total number of hydroxy groups assigned to first polyhydric alcohol-derived units (namely, the total number of hydroxy groups of the first polyhydric alcohol to constitute the first polyhydric alcohol-derived units) to the total number of units derived from the single type of monomer in the second polyhydric alcohol-derived units or in the second polyhydric alcohol. This configuration is advantageous for balancing between the technical effects by the first polyhydric alcohol-derived units and the technical effects by the second polyhydric alcohol-derived units. In addition, the configuration is advantageous for restraining excessive hydrophilicity of the polyester-polyether polyurethane in the pressure-sensitive adhesive layer, consequently, restraining excessive hydrophilicity of the pressure-sensitive adhesive layer, and advantageous for allowing the adhesive face in the identification sheet to surely collect a hydrophobic component (such as an oil component in a secretion that forms fingerprints).

Preferably, the multifunctional isocyanate is an aliphatic or alicyclic multifunctional isocyanate. This configuration is advantageous for allowing the pressure-sensitive adhesive layer in the identification sheet to actually have high transparency. More preferably, the multifunctional isocyanate is at least one selected from the group consisting of hexamethylene diisocyanate, 1,3-bis(isocyanatomethyl)cyclohexane, isophorone diisocyanate, 4,4'-methylenebis(cyclohexyl isocyanate), and hydrogenated diphenylmethane diisocyanate. Among them, hexamethylene diisocyanate is particularly preferred as the multifunctional isocyanate, for allowing the polyester polyurethane to actually have not only high transparency, but also appropriate cohesiveness, and, consequently, for allowing the pressure-sensitive adhesive layer in the identification sheet to actually have not only high transparency, but also appropriate cohesiveness. This is probably because hexamethylene diisocyanate gives a polyester polyurethane having appropriate balance between the number of isocyanate groups to constitute hydrophilic urethane bonds and the number of methylene groups to constitute a hydrophobic skeleton, where the balance is appropriate for the polyester polyurethane when used to constitute the pressure-sensitive adhesive layer for use in the identification sheet.

Preferably, the polycarboxylic acid is an aliphatic or alicyclic polycarboxylic acid. This configuration is advantageous for allowing the pressure-sensitive adhesive layer in the identification sheet to actually have high transparency. More preferably, the polycarboxylic acid is adipic acid. Assume that the polyester polyurethane includes units derived from hexamethylene diisocyanate (first units) and units derived from adipic acid (second units). In this case, a pair of urethane bonds and a pair of ester bonds respectively in each first unit and in each second unit tend to form two pairs of hydrogen bonds. This is because the distance between the pair of urethane bonds in the first unit approximates to the distance between the pair of ester bonds in the second unit. The interaction as above is usable as one of approaches to control the cohesiveness of the polyester polyurethane. Thus, the combination use of hexamethylene diisocyanate and adipic acid as monomers to form the polyester polyurethane is preferred from the viewpoint of controlling the cohesiveness of the polyester polyurethane, or of the pressure-sensitive adhesive layer containing the polyester polyurethane.

Preferably, the first polyhydric alcohol is an aliphatic or alicyclic polyhydric alcohol. This configuration is advantageous for allowing the pressure-sensitive adhesive layer in the identification sheet to actually have high transparency. More preferably, the first polyhydric alcohol is neopentyl glycol. Neopentyl glycol, which has a branched chain structure, is expected to have a higher action of relaxing or lowering the cohesiveness between polyester polyurethane molecular chains when the neopentyl glycol is incorporated in the polyester polyurethane, as compared with linear polyols. The cohesiveness lowering action as above is usable as one of approaches to control the cohesiveness of the polyester polyurethane. Accordingly, the use of neopentyl glycol as a polyol component to form the polyester polyurethane is preferred from the viewpoint of controlling the cohesiveness of the polyester polyurethane, or of the pressure-sensitive adhesive layer containing the polyester polyurethane.

Preferably, the second polyhydric alcohol is an aliphatic or alicyclic polyhydric alcohol containing an ether bond. This configuration is advantageous for allowing the pressure-sensitive adhesive layer in the identification sheet to actually have high transparency. More preferably, the second polyhydric alcohol is a polypropylene glycol. Such a polypropylene glycol not only has high flexibility, but also is readily designable to have multiple functions and/or to have a high molecular weight. Thus, the use of the polypropylene glycol as a polyol component to form the polyester-polyether polyurethane is advantageous for providing high degree of freedom in molecular design of the polyester-polyether polyurethane while imparting flexibility to the polyester-polyether polyurethane.

Preferably, for the polyester-polyether polyurethane in the pressure-sensitive adhesive layer, the multifunctional isocyanate is hexamethylene diisocyanate, the polycarboxylic acid is adipic acid, the first polyhydric alcohol is neopentyl glycol, and the second polyhydric alcohol is a polypropylene glycol, where units or segments of the polyester-polyether polyurethane are derived from these substances.

The polyester polyurethane, which serves as a pressure-sensitive adhesive, in the pressure-sensitive adhesive layer has a softening temperature of preferably 190°C to 280°C. The softening temperature of the pressure-sensitive adhesive may be measured typically using a thermomechanical analyzer TMA/SS7100 (trade name, supplied by SII NanoTechnology Inc.). In this measurement, for example, a test piece (6 mm by 6 mm) to be subjected to the measurement is cut out from a sample pressure-sensitive adhesive sheet having a multilayer structure including a poly(ethylene terephthalate) substrate and a pressure-sensitive adhesive layer (thickness: 290 µm). The measurement is performed in a penetration mode using a needle penetrator probe having a tip diameter of 1 mm under a load of 1470 mN, in which the tip of the probe is brought in contact with the central part of the pressure-sensitive adhesive surface of the test piece. In the measurement, the temperature is raised from a start temperature of 150°C up to an end temperature of 320°C at a rate of temperature rise of 10°C/min, in a nitrogen stream at a flow rate of 200 ml/min.

Preferably, the pressure-sensitive adhesive sheet in the identification sheet has a multilayer structure including the pressure-sensitive adhesive layer and a substrate layer; and, with this, the identification sheet further includes a backing sheet having a mount face to which the pressure-sensitive adhesive layer of the pressure-sensitive adhesive sheet is removably attachable. This configuration is advantageous from the viewpoint of handleability of the identification sheet.

In the identification sheet including the backing sheet and the pressure-sensitive adhesive sheet, both of which are removable or separable from each other, preferably, the backing sheet has a first outer face on opposite side to the mount face, where the first outer face bears a first distinguishing mark; and the pressure-sensitive adhesive sheet has a second outer face in the substrate layer on opposite side to the pressure-sensitive adhesive layer, where the second outer face bears a second distinguishing mark.

In the identification sheet, when having the configuration as above, the first distinguishing mark disposed at the first outer face defined by the backing sheet is usable as first distinguishing information, and the second distinguishing mark disposed at the second outer face defined by the pressure-sensitive adhesive sheet is usable as second distinguishing information. The first outer face bearing the first distinguishing mark may be covered with a transparent protective film. The second outer face bearing the second distinguishing mark may be covered with a transparent protective film. The first and second distinguishing marks in the identification sheet can be configured to function as a pair of cross-check information to determine whether a backing sheet with the first distinguishing mark and a pressure-sensitive adhesive sheet with the second distinguishing mark correspond to each other, by designing the two distinguishing marks to correspond to each other. The first and second distinguishing marks that function as a pair of cross-check information may have pattern shapes identical to, or different from, each other. The first and second distinguishing marks each independently include at least one selected typically from numeric characters, alphabetic characters, hiragana characters, katakana characters, kanji characters, symbols, bar codes, and QR Codes. Examples of the bar codes include one-dimensional bar codes and two-dimensional bar codes.

Using the identification sheet having the configuration as above, a target material (material to be identified) may be collected typically by the following procedure. The pressure-sensitive adhesive sheet having the second outer face with the second distinguishing mark is removed from the mount face of the backing sheet having the first outer face with the first distinguishing mark. The target material is then attached to the exposed pressure-sensitive adhesive layer or the adhesive face of the pressure-sensitive adhesive sheet. The pressure-sensitive adhesive sheet with the second distinguishing mark, which bears the target material, is laminated again onto the mount face of the original backing sheet with the first distinguishing mark. In the lamination, the adhesive face side, of the transparent pressure-sensitive adhesive sheet, bearing the target material is attached onto the mount face. On a writable surface, which may be provided in a predetermined area of the backing sheet, data relating to the collection of the target material are recorded or written, and a person such as a collector signs and seals the backing sheet on its writable surface. Non-limiting examples of the data include date, place, collected material name, and reference number. In the identification sheet, which is used typically in the above manner, the first distinguishing mark of the backing sheet and the second distinguishing mark of the pressure-sensitive adhesive sheet correspond to each other and act as a pair in the single identification sheet. This guarantees that the backing sheet and the pressure-sensitive adhesive sheet correspond to each other. Assume that, in the identification sheet after the collection of the target material, the pressure-sensitive adhesive sheet bearing the true target material and adhering to the backing sheet is removed and replaced with another pressure-sensitive adhesive sheet, where the other pressure-sensitive adhesive sheet is a pressure-sensitive adhesive sheet that lacks the second distinguishing mark corresponding to the first distinguishing mark of the backing sheet. In this case, there can be determined the absence of mutual correspondence between the backing sheet and the pressure-sensitive adhesive sheet after replacement (the other pressure-sensitive adhesive sheet). This is because the other pressure-sensitive adhesive sheet lacks the second distinguishing mark corresponding to the first distinguishing mark of the backing sheet. Assume that, in the identification sheet after the collection of the target material, the backing sheet, to which the pressure-sensitive adhesive sheet bearing the true target material adheres, and on which the data such as various information for the target material are recorded, is removed and replaced with another backing sheet, where the other backing sheet is a backing sheet that lacks the first distinguishing mark corresponding to the second distinguishing mark of the pressure-sensitive adhesive sheet. In this case, there can be determined the absence of mutual correspondence between the pressure-sensitive adhesive sheet and the backing sheet after replacement (the other backing sheet). This is because the other backing sheet lacks the first distinguishing mark corresponding to the second distinguishing mark of the pressure-sensitive adhesive sheet. Specifically, the identification sheet includes the backing sheet and the pressure-sensitive adhesive sheet, where the backing sheet bears the first distinguishing mark, which is one of the pair of cross-check information, and the pressure-sensitive adhesive sheet bears the second distinguishing mark, which is the other of the pair of cross-check information. If the identification sheet undergoes such a replacement as above, the replacement can be detected or recognized. The identification sheet is therefore suitable for restraining a tampering act on the identification sheet after the collection of the target material and for restraining the mistaking of the pressure-sensitive adhesive sheet or the backing sheet for another one during a collecting process and an identifying operation for the target material. Consequently, the identification sheet is suitable for surely providing the admissibility of evidence required of the target material collected typically in a criminal case.

In addition, in the identification sheet, the first distinguishing mark has been formed at the first outer face of the backing sheet, separately from the second distinguishing mark; and the second distinguishing mark has been formed at the second outer face of the pressure-sensitive adhesive sheet, separately from the first distinguishing mark. The first distinguishing mark in the identification sheet is neither one formed by copying as a result of a transferring action of a coloring component constituting part of another distinguishing mark; nor one formed by copying as a result of a physical and/or chemical reaction caused typically by local heating, pressurization, or photoirradiation in the formation of another distinguishing mark. The second distinguishing mark in the identification sheet is neither one formed by copying as a result of a transferring action of a coloring component constituting part of another distinguishing mark; nor one formed by copying as a result of a physical and/or chemical reaction caused typically by local heating, pressurization, or photoirradiation in the formation of another distinguishing mark. The first and second distinguishing marks are ones separately or individually formed or engraved in the identification sheet. This configuration is advantageous for restraining or avoiding deterioration of the first and second distinguishing marks and is therefore suitable for allowing the two distinguishing marks to keep on functioning as a pair of cross-check information so as to determine the mutual correspondence between a backing sheet with the first distinguishing mark and a pressure-sensitive adhesive sheet with the second distinguishing mark.

As described above, the identification sheet having the configuration with the first and second distinguishing marks is suitable for restraining a tampering act on the identification sheet after the collection of the target material and for restraining mistaking of the pressure-sensitive adhesive sheet or the backing sheet for another one during a collecting process and an identifying operation for the target material. Consequently, the identification sheet is suitable for keeping on surely providing the admissibility of evidence of the collected target material, where the admissibility of evidence is required typically in a criminal case.

Preferably, the first distinguishing mark and the second distinguishing mark are each independently selected from an engraved mark and a printed mark. As used herein, the term "engraved mark" refers to a mark which is made by engraving to form a concave in a face to be marked. Non-limiting examples of the engraving technique include laser marking and marking with air pen (air pen marking). More preferably, the first distinguishing mark and the second distinguishing mark are each independently selected from a laser mark, which is an engraved mark formed by laser marking; and a mark resulting from air pen marking, which is an engraved mark formed by air pen marking. These configurations are suitable for restraining or avoiding deterioration of the first and second distinguishing marks. The configurations are therefore advantageous for allowing the two distinguishing marks to keep on functioning as a pair of cross-check information to determine the mutual correspondence between a backing sheet with the first distinguishing mark and a pressure-sensitive adhesive sheet with the second distinguishing mark. Such an engraved mark is advantageous for giving a face to be marked, which less undergoes a shape change (such as formation of scratch marks) other than the formed distinguishing mark. In addition, the laser mark, which is a mark formed by laser marking, is also advantageous for imparting high degree of freedom to designing or determination of the pattern shape of the distinguishing mark. This contributes to better productivity of the identification sheet.

The engraved mark formed by laser marking is also advantageous for surely providing removability or releasability between the backing sheet and the pressure-sensitive adhesive sheet. Assume that, in an identification sheet including a backing sheet and a pressure-sensitive adhesive sheet laminated on each other, a laser mark is formed as a through hole penetrating the backing sheet by laser irradiation from the backing sheet side, or a laser mark is formed as a through hole penetrating the pressure-sensitive adhesive sheet by laser irradiation from the pressure-sensitive adhesive sheet side, or a laser mark is formed as a through hole penetrating the entire identification sheet by laser irradiation. In these cases, the pressure-sensitive adhesive is softened and melted at or around an area, in which the through hole is formed, in a surface of the pressure-sensitive adhesive layer of the pressure-sensitive adhesive sheet, where the surface faces the backing sheet. Thus, the pressure-sensitive adhesive layer is liable to fuse to the backing sheet in the area. The fusion, if occurring, may impede the separation, namely, the removal between the backing sheet and the pressure-sensitive adhesive sheet, and/or may cause the pressure-sensitive adhesive layer to be significantly damaged, because the pressure-sensitive adhesive layer at the fused portion is pulled by the backing sheet upon removal (separation). In the configuration, the identification sheet includes an engraved mark formed by laser marking as at least one of the first distinguishing mark and the second distinguishing mark. This configuration is advantageous for avoiding or minimizing the occurrence of local fusion between the backing sheet and the pressure-sensitive adhesive sheet and for surely providing the removability between the two sheets. Softening and melting of the pressure-sensitive adhesive in the adhesive face causes deformation of the softened-melted portion in the pressure-sensitive adhesive layer. The resulting deformation or unevenness in the adhesive face tends to lower the adhesion between the backing sheet and the pressure-sensitive adhesive sheet, when the two sheets after removal are laminated again on each other. The configuration, where the identification sheet includes the engraved mark formed by laser marking as at least one of the first distinguishing mark and the second distinguishing mark, is advantageous for avoiding or minimizing such deformation and unevenness in the adhesive face, and for surely providing the adhesion between the backing sheet and the pressure-sensitive adhesive sheet after re-lamination.

### Brief Description of Drawings

FIG. 1 is a plan view of an identification sheet according to one embodiment of the present invention;
FIG. 2 is another plan view of the identification sheet illustrated in FIG. 1;
FIG. 3 is a partial enlarged cross-sectional view of the identification sheet illustrated in FIG. 1;
FIG. 4 illustrates a configuration in which two or more of the identification sheet illustrated in FIG. 1 are unified;
FIG. 5 is a plan view of one modification of the identification sheet illustrated in FIG. 1;
FIG. 6 is another plan view of the identification sheet illustrated in FIG. 5;
FIG. 7 is a partial enlarged cross-sectional view of the identification sheet illustrated in FIG. 5;
FIG. 8 illustrates a configuration in which two or more of the identification sheet illustrated in FIG. 5 are unified; and
FIG. 9 is a partial enlarged cross-sectional view of one modification of the identification sheet illustrated in FIG. 1.

### Description of Embodiments

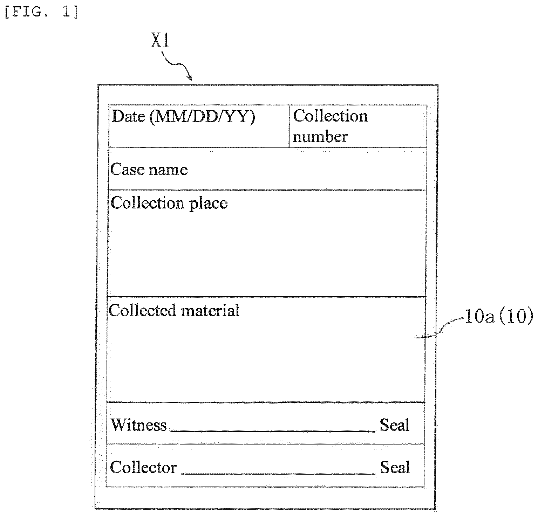

FIGs. 1 to 3 illustrate an identification sheet X1 according to one embodiment of the present invention. FIGs. 1, 2, and 3 are a plan view, another plan view, and a partial enlarged cross-sectional view, respectively, of the identification sheet X1.

The identification sheet X1 includes a backing sheet 10 and a pressure-sensitive adhesive sheet 20, which are separable from each other. The identification sheet X1 has an outer face 10a and an outer face 20a. The outer face 10a can serve as an outer surface defined by the backing sheet 10. The outer face 20a can serve as an outer surface defined by the pressure-sensitive adhesive sheet 20.

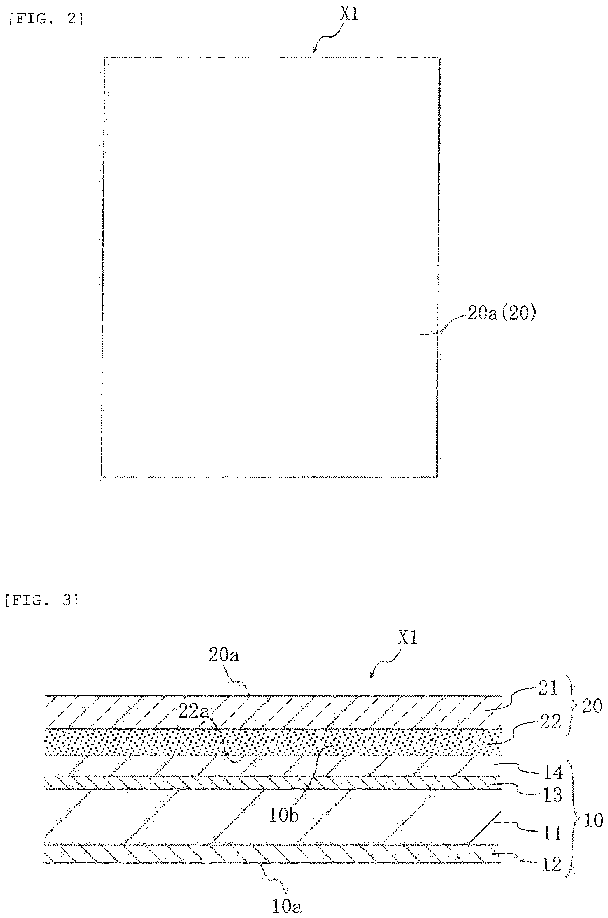

The backing sheet 10 has a multilayer structure including a base layer 11, a writable layer 12, a background layer 13, and a release layer 14, as illustrated in FIG. 3.

The base layer 11 of the backing sheet 10 is a portion that functions as a support or substrate in the backing sheet 10. Examples of a material or materials to constitute the base layer 11 include, but are not limited to, synthetic paper, woodfree paper, Kent paper, polyester films, and polyolefin films. Non-limiting examples of the polyester films include poly(ethylene terephthalate) films and poly(butylene terephthalate) films. Non-limiting examples of the polyolefin films include polyethylene films and polypropylene films. The base layer 11 has a thickness of typically 2.5 to 500 µm, preferably 25 to 300 µm, more preferably 50 to 200 µm, and furthermore preferably 100 to 150 µm.

The writable layer 12 of the backing sheet 10 is a portion that provides a writable surface. The outer surface defined by the writable layer 12 defines or constitutes the outer face 10a, which can be an outer surface of the backing sheet 10 or the identification sheet X1. In this embodiment, the outer surface is a writable surface of the identification sheet X1 or the backing sheet 10. The writable surface provided by the writable layer 12 includes at least one preprinted box in which a set of information relating to collection of a target material is, for example, to be recorded, as illustrated in FIG. 1. Each preprinted box typically includes blanks for a date, for a collection number, for a case name, for a collection place, for a collected material name (collected item name), for a collector's signature and seal, and for a witness's signature and seal. The preprinted box as above is formed typically by printing on the writable layer 12. Non-limiting examples of a material or materials to constitute the writable layer 12 include polyurethane inks, polyester inks, acrylic inks, and polystyrene inks, each of which is colored white or whitish. The material(s) to constitute the writable layer 12 is preferably selected from polyurethane inks, from the viewpoint of providing high heat resistance of the writable layer 12. For example, assume that the outer face 10a of the identification sheet X1 is further subjected to processing with heating. In this case, the higher the heat resistance of the material to constitute the writable layer is, the better. The writable layer 12 as above has a thickness of typically 0.01 to 100 µm, preferably 0.1 to 30 µm, and more preferably 0.5 to 10 µm.

The background layer 13 of the backing sheet 10 is a colored layer and is typically colored black or white. Non-limiting examples of a material or materials to constitute the background layer 13 include polyurethane inks, polyester inks, acrylic inks, and polystyrene inks. The background layer 13 as above has a thickness of typically 0.01 to 100 µm, preferably 0.1 to 50 µm, and more preferably 0.5 to 30 µm.

The release layer 14 of the backing sheet 10 is a layer that surely offers releasability (removability) between the backing sheet 10 and the pressure-sensitive adhesive sheet 20 and is typically transparent. A non-limiting example of a material or materials to constitute the release layer 14 is a transparent plastic film. Non-limiting examples of the plastic film include polyethylene films and poly(ethylene terephthalate) films. The release layer 14 has a thickness of typically 2.5 to 500 µm, preferably 10 to 250 µm, and more preferably 20 to 125 µm. In the release layer 14 as above, the surface facing the pressure-sensitive adhesive sheet 20 defines a mount face 10b of the backing sheet 10.

The pressure-sensitive adhesive sheet 20 of the identification sheet X1 has a multilayer structure including a substrate layer 21 and a pressure-sensitive adhesive layer 22, as illustrated in FIG. 3.

The substrate layer 21 of the pressure-sensitive adhesive sheet 20 is a portion that serves as a carrier (support) in the pressure-sensitive adhesive sheet 20. The outer surface defined by the substrate layer 21 constitutes the outer face 20a, which can serve as one outer surface of the pressure-sensitive adhesive sheet 20 or the identification sheet X1. The higher the transparency of the substrate layer 21 is, the more preferred from the viewpoint of surely offering visibility of a target material when seen from the pressure-sensitive adhesive sheet 20 side, where the target material is to be held between the backing sheet 10 and the pressure-sensitive adhesive sheet 20 in the identification sheet X1. Non-limiting examples of a material or materials to constitute the substrate layer 21 as above include polyester films and polyolefin films. Non-limiting examples of the polyester films include poly(ethylene terephthalate) films and poly(butylene terephthalate) films. Non-limiting examples of the polyolefin films include polyethylene films and polypropylene films. The constituent material of the substrate layer 21 is preferably selected from poly(ethylene terephthalate) films, from the viewpoint of allowing the substrate layer 21 to have high transparency. The substrate layer 21 as above has a thickness of preferably 2.5 to 500 µm, more preferably 25 to 250 µm, and furthermore preferably 50 to 125 µm.

The pressure-sensitive adhesive layer 22 of the pressure-sensitive adhesive sheet 20 has an adhesive face 22a and is removably attachable to the mount face 10b of the backing sheet 10. The pressure-sensitive adhesive layer 22 is formed from a pressure-sensitive adhesive composition containing, as a principal component (base polymer), a polyester polyurethane, which serves as a pressure-sensitive adhesive. As used herein, the term "principal component" refers to a component that occupies the largest weight proportion among components contained therein. The higher the transparency of the pressure-sensitive adhesive layer 22 is, the more preferred from the viewpoint of surely offering visibility of a target material when seen from the pressure-sensitive adhesive sheet 20 side, where the target material is to be held between the backing sheet 10 and the pressure-sensitive adhesive sheet 20 in the identification sheet X1. The pressure-sensitive adhesive layer 22 as above has a storage modulus of typically 3× 10³ to 9× 10⁶ Pa, preferably 3× 10³ to 9× 10⁵ Pa, and more preferably 3× 10³ to 9× 10⁴ Pa. The pressure-sensitive adhesive layer 22 has an adhesive strength of typically 0.01 to 10 N/20 mm, preferably 0.03 to 5 N/20 mm, more preferably 0.05 to 2 N/20 mm, and furthermore preferably 0.07 to 0.7 N/20 mm. The pressure-sensitive adhesive layer 22 has a thickness of typically 10 to 1000 µm, more preferably 150 to 800 µm, and furthermore preferably 200 to 500 µm.

The polyester polyurethane in the pressure-sensitive adhesive layer 22 is a polymer containing many urethane bonds and many ester bonds in its molecular chains and is a polymer derived typically from a polyhydric alcohol, a multifunctional isocyanate, and a polycarboxylic acid or an ester thereof, optionally in combination with an active hydrogen-containing compound. Non-limiting examples of the polyhydric alcohol include polymer glycols and low-molecular glycols. Non-limiting examples of the multifunctional isocyanate include diisocyanates. Non-limiting examples of the polycarboxylic acid include dicarboxylic acids. The polyester polyurethane can be synthetically prepared typically by solution polymerization, emulsion polymerization, or bulk polymerization, according to a so-called prepolymer process, which is a two-step synthesis, or according to a so-called one-shot process, which is a one-step synthesis.

The urethane bonds of the polyester polyurethane are hydrogen-bonding functional groups each containing both a hydrogen donor group and a hydrogen acceptor group. The polyester polyurethane, which contains the hydrogen-bonding functional groups as above, can efficiently form hydrogen bonds between its molecular chains to cohere with each other and has relatively high cohesive energy. This tends to allow the polyester polyurethane to actually have a high softening temperature and/or a high glass transition temperature, and, consequently high heat resistance. The polyester polyurethane, which is contained as a pressure-sensitive adhesive in the pressure-sensitive adhesive layer 22, has a softening temperature of preferably 190°C to 280°C, more preferably 200°C to 270°C, and still more preferably 210°C to 260°C. The softening temperature of the pressure-sensitive adhesive can be measured typically using a thermomechanical analyzer TMA/SS7100 (trade name, supplied by SII NanoTechnology Inc.). In this measurement, for example, a test piece (6 mm by 6 mm) is prepared by cutting out from a sample pressure-sensitive adhesive sheet having a multilayer structure including a poly(ethylene terephthalate) substrate and a pressure-sensitive adhesive layer (thickness: 290 µm). The measurement is performed in a penetration mode using a needle penetrator probe having a tip diameter of 1 mm under a load of 1470 mN, in which the tip of the probe is brought in contact with the central part of the pressure-sensitive adhesive surface of the test piece. In the measurement, the temperature is raised from a start temperature of 150°C up to an end temperature of 320°C at a rate of temperature rise of 10°C/min, in a nitrogen stream at a flow rate of 200 ml/min. The glass transition temperature of the pressure-sensitive adhesive may be measured by differential scanning calorimetry (DSC) in conformity to JIS K 7121: Testing Methods for Transition Temperatures of Plastics. The configuration, where such a polyester polyurethane as to readily actually offer high heat resistance is contained as a principal component in the pressure-sensitive adhesive layer 22, tends to allow the pressure-sensitive adhesive layer 22 in the identification sheet X1 to actually have high heat resistance. The pressure-sensitive adhesive layer 22, with increasing heat resistance thereof, more resists or less undergoes thermal deformation.

The polyester polyurethane contains not only urethane bonds as described above, but also ester bonds, which are hydrogen acceptor groups. In the polyester polyurethane, the ester bonds less cause molecular chains to cohere with each other, as compared with the urethane bonds. The regulation of the content or proportion of ester bonds in the polyester polyurethane is usable as one of approaches to control the cohesiveness of the polyester polyurethane. This allows the polyester polyurethane, which contains not only urethane bonds, but also ester bonds as above, tends to be controllable on cohesive force between molecular chains, within such a range as to offer sufficiently high heat resistance in the pressure-sensitive adhesive layer 22 for use in the identification sheet. The control of the cohesiveness of the pressure-sensitive adhesive layer 22 contributes to allowing the pressure-sensitive adhesive layer 22 to actually have good adhesive strength and to less leave adhesive residue on an adherend after removal of the pressure-sensitive adhesive sheet 20 or the pressure-sensitive adhesive layer 22 from the adherend. The identification sheet X1 tends to allow the pressure-sensitive adhesive layer 22 to actually have high heat resistance while controlling the cohesiveness of the pressure-sensitive adhesive layer 22.

In addition, the polyester polyurethane can be formed from material monomers selectable from among a wide variety of practically easily available high-purity monomers. With increasing purities of material monomers, a polymer resulting from the material monomers tends to have higher transparency. Accordingly, such a polyester polyurethane, which can be formed from material monomers selectable from among a wide variety of practically easily available high-purity monomers, tends to actually have high transparency. The configuration, where the polyester polyurethane is contained as a principal component in the pressure-sensitive adhesive layer 22, tends to allow the pressure-sensitive adhesive layer 22 in the identification sheet X1 to actually have high transparency. Assume that the identification sheet X1 is produced by a process through a step in which laser beams for processing typically pass through the pressure-sensitive adhesive layer 22. In this case, the pressure-sensitive adhesive layer 22, with increasing transparency thereof, less absorbs laser beams and less undergoes thermal deformation due to laser beam absorption.

As described above, the identification sheet X1 includes the pressure-sensitive adhesive layer 22, which adequately resists thermal deformation. In addition, the configuration, where the polyester polyurethane, which tends to actually have a high softening temperature and/or a high glass transition temperature as described above, is contained as a principal component in the pressure-sensitive adhesive layer 22, tends to allow the pressure-sensitive adhesive layer 22 to actually have high thermal stability and/or low fluidity. The high thermal stability and/or low fluidity of the pressure-sensitive adhesive layer 22 in the identification sheet X1 is advantageous for long-term, stable storage of the target material held by the identification sheet X1.

The polyester polyurethane in the pressure-sensitive adhesive layer 22 preferably includes units derived from a multifunctional isocyanate, units derived from a polycarboxylic acid, and units derived from a first polyhydric alcohol. This configuration can appropriately give the polyester polyurethane with the technical effects.

The polyester polyurethane in the pressure-sensitive adhesive layer 22 is preferably a polyester-polyether polyurethane. Such a polyester-polyether polyurethane contains a polyether skeleton, in addition to urethane bonds and ester bonds as described above. Ether bonds contained in the polyether skeleton have low optical absorptivity and have higher flexibility or mobility as compared with carbon-carbon-carbon bonds. The polyester-polyether polyurethane, which contains such a polyether skeleton in addition to urethane bonds and ester bonds, tends to actually have high transparency and good flexibility, while controlling the cohesive force between its molecular chains within such a range as to impart sufficiently high heat resistance to the pressure-sensitive adhesive layer 22 for use in the identification sheet. Polyester polyurethanes, to which the polyester-polyether polyurethane belongs, can be formed from material monomers which are selectable from among a wide variety of actually easily available high-purity monomers. The polyester-polyether polyurethane, which belongs to polyester polyurethanes capable of being made from material monomers selectable from among a wide variety of easily available high-purity material monomers, tends to actually have high transparency. The configuration, where the polyester-polyether polyurethane as above is contained as a principal component in the pressure-sensitive adhesive layer 22, tends to allow the pressure-sensitive adhesive layer 22 in the identification sheet X1 not only to actually have high heat resistance with the control of cohesiveness, but also actually have both high transparency and good flexibility. The pressure-sensitive adhesive layer 22, with increasing heat resistance thereof, less thermally deforms, as described above. The pressure-sensitive adhesive layer 22, with increasing transparency thereof, less thermally deforms, where the thermal deformation will be caused by laser beam absorption, as described above. With increasing flexibility of the pressure-sensitive adhesive layer 22, the adhesive face 22a of the pressure-sensitive adhesive layer 22 more conforms to, and more deforms with, the surface asperities of a target material when the pressure-sensitive adhesive layer 22 or the adhesive face 22a is pressed onto the target material, and the asperities of the target material are more readily transferred onto the pressure-sensitive adhesive layer 22. Balancing between flexibility and thermal stability-low fluidity of the pressure-sensitive adhesive layer 22 in the identification sheet X1 can allow the pressure-sensitive adhesive layer 22 or the adhesive face 22a to actually have such tackiness as to adhere to the target material with good conformity to the asperities and can still less cause so-called stringiness of the pressure-sensitive adhesive between an adherend and the adhesive face 22a upon removal of the pressure-sensitive adhesive layer 22 from the adherend.

The polyester-polyether polyurethane, when contained as a pressure-sensitive adhesive in the pressure-sensitive adhesive layer 22, preferably includes units derived from a multifunctional isocyanate, units derived from a polycarboxylic acid, units derived from a first polyhydric alcohol, and units derived from a second polyhydric alcohol containing an ether bond. This configuration can appropriately give the polyester-polyether polyurethane with the technical effects.

Non-limiting examples of the multifunctional isocyanate include hexamethylene diisocyanate (HDI), 1,3-bis(isocyanatomethyl)cyclohexane, isophorone diisocyanate, 4,4'-methylenebis(cyclohexyl isocyanate), diphenylmethane diisocyanate (MDI), tolylene diisocyanate (TDI), xylylene diisocyanate (XDI), hydrogenated MDI, and TDI adducts. The multifunctional isocyanate is preferably selected from aliphatic or alicyclic multifunctional isocyanates. This configuration is advantageous for allowing the pressure-sensitive adhesive layer 22 in the identification sheet X1 to actually have high transparency. More preferably, the multifunctional isocyanate is at least one selected from the group consisting of hexamethylene diisocyanate, 1,3-bis(isocyanatomethyl)cyclohexane, isophorone diisocyanate, 4,4'-methylenebis(cyclohexyl isocyanate), and hydrogenated MDI. Among them, hexamethylene diisocyanate is particularly preferred as the multifunctional isocyanate. This is preferred for allowing the polyester polyurethane to actually have not only high transparency, but also appropriate cohesiveness, and consequently for allowing the pressure-sensitive adhesive layer 22 in the identification sheet X1 to actually have not only high transparency, but also appropriate cohesiveness. This is probably because hexamethylene diisocyanate gives a polyester polyurethane having appropriate balance between the number of isocyanate groups to constitute hydrophilic urethane bonds and the number of methylene groups to constitute a hydrophobic skeleton, where the balance is appropriate for the polyester polyurethane when used to constitute the pressure-sensitive adhesive layer 22 for use in the identification sheet.

Examples of the polycarboxylic acid include, but are not limited to, aliphatic or alicyclic polycarboxylic acids and aromatic polycarboxylic acids. Non-limiting examples of the aliphatic or alicyclic polycarboxylic acids include malonic acid, succinic acid, glutaric acid, adipic acid, and sebacic acid. Non-limiting examples of the aromatic polycarboxylic acids include terephthalic acid and trimellitic acid. The polycarboxylic acid is preferably selected from aliphatic or alicyclic polycarboxylic acids. This configuration is advantageous for allowing the pressure-sensitive adhesive layer 22 in the identification sheet X1 to actually have high transparency. More preferably, the polycarboxylic acid is adipic acid. Assume that the polyester polyurethane includes units derived from hexamethylene diisocyanate (first units) and units derived from adipic acid (second units). In this case, a pair of urethane bonds and a pair of ester bonds respectively in each first unit and in each second unit tend to form two pairs of hydrogen bonds. This is because the distance between the pair of urethane bonds in the first unit approximates to the distance between the pair of ester bonds in the second unit. The interaction as above is usable as one of approaches to control the cohesiveness of the polyester polyurethane. Thus, the combination use of hexamethylene diisocyanate and adipic acid as monomers to form the polyester polyurethane is preferred from the viewpoint of controlling the cohesiveness of the polyester polyurethane or of the pressure-sensitive adhesive layer 22 containing the polyester polyurethane.

Examples of the first polyhydric alcohol include, but are not limited to, aliphatic or alicyclic polyhydric alcohols and aromatic polyhydric alcohols. Non-limiting examples of the aliphatic or alicyclic polyhydric alcohols include ethylene glycol, propylene glycol, butylene glycol, neopentyl glycol, and cyclohexanediol. Non-limiting examples of the aromatic polyhydric alcohols include bisphenol-A and resorcinol. The first polyhydric alcohol is preferably selected from aliphatic or alicyclic polyhydric alcohols. This configuration is advantageous for allowing the pressure-sensitive adhesive layer 22 in the identification sheet X1 to actually have high transparency. More preferably, the first polyhydric alcohol is neopentyl glycol. Neopentyl glycol, which has a branched chain structure, is expected to have a higher action of relaxing or lowering the cohesiveness between polyester polyurethane molecular chains when the neopentyl glycol is incorporated in the polyester polyurethane, as compared with linear polyols. The cohesiveness lowering action as above is usable as one of approaches to control the cohesiveness of the polyester polyurethane. Accordingly, the use of neopentyl glycol as a polyol component to form the polyester polyurethane is preferred from the viewpoint of controlling the cohesiveness of the polyester polyurethane or of the pressure-sensitive adhesive layer 22 containing the polyester polyurethane.

The second polyhydric alcohol is preferably selected from aliphatic or alicyclic polyhydric alcohols containing an ether bond. The aliphatic or alicyclic polyhydric alcohols containing an ether bond may be ones that are crosslinked typically with glycerol or ethylene glycol. Non-limiting examples of the second polyhydric alcohol include polyethylene glycols and polypropylene glycols. The second polyhydric alcohol as above has a number average molecular weight of preferably 300 to 50000, more preferably 500 to 10000, furthermore preferably 500 to 5000, still more preferably 500 to 4000, and still furthermore preferably 1000 to 3000. The "number average molecular weight" herein is a value obtained by gel permeation chromatographic (GPC) measurement and calibration with a polyethylene oxide (PEO) as a standard polymer sample. The configuration as above relating to the second polyhydric alcohol is advantageous for allowing the pressure-sensitive adhesive layer 22 in the identification sheet X1 to actually have high transparency. More preferably, the second polyhydric alcohol is a polypropylene glycol. The polypropylene glycol not only has high flexibility, but also is readily designable to have multiple functions and/or to have a high molecular weight. Thus, the use of the polypropylene glycol as a polyol component to form the polyester-polyether polyurethane is advantageous for providing high degree of freedom in molecular design of the polyester-polyether polyurethane, while imparting flexibility to the polyester-polyether polyurethane.

In the polyester polyurethane (including the polyester-polyether polyurethane) in the pressure-sensitive adhesive layer 22, a first ratio is preferably 0.5 to 1.5, more preferably 0.7 to 1.4, furthermore preferably 0.7 to 1.3, and still more preferably 0.8 to 1.2, where the first ratio is the ratio of the total number of hydroxy groups in the polyhydric alcohol(s) (namely, the total number of hydroxy groups in the polyhydric alcohol(s) to constitute polyhydric alcohol-derived units) to the sum of the total number of isocyanate groups in the multifunctional isocyanate (namely, the total number of isocyanate groups in the multifunctional isocyanate to constitute multifunctional isocyanate-derived units) and the total number of carboxy groups in the polycarboxylic acid (namely, the total number of carboxy groups in the polycarboxylic acid to constitute polycarboxylic acid-derived units). With the ratio (first ratio) approaching 1, the polyester polyurethane tends to have a higher molecular weight and, due to such a high molecular weight, tends to offer higher cohesiveness between molecules of the polyester polyurethane. With the ratio decreasing less than 1, isocyanate groups and carboxy groups tend to be present in a total number larger than the number of hydroxy groups in entire molecular ends of the polyester polyurethane, and, accordingly, the cohesiveness between molecules of the polyester polyurethane tends to be higher, where the cohesiveness is due to the presence of isocyanate groups and carboxy groups, both of which are highly polar groups which relatively highly polarize. With the ratio increasing greater than 1, hydroxy groups tend to be present in a number larger than the total number of isocyanate groups and carboxy groups in entire molecular ends of the polyester polyurethane, where the hydroxy groups have lower optical absorptivity as compared with the isocyanate groups and the carboxy groups. In this case, the polyester polyurethane therefore tends to have higher transparency. In other words, in the polyester polyurethane in the pressure-sensitive adhesive layer 22, the first ratio is preferably 0.5 to 1.5, more preferably 0.7 to 1.4, furthermore preferably 0.7 to 1.3, and still more preferably 0.8 to 1.2, from the viewpoints of balancing between cohesiveness and transparency of the polyester polyurethane, and, consequently, from the viewpoint of balancing between cohesiveness and transparency of the pressure-sensitive adhesive layer 22 containing the polyester polyurethane, where the first ratio is the ratio of the total number of hydroxy groups in the polyhydric alcohol(s) to the sum of the total number of isocyanate groups in the multifunctional isocyanate and the total number of carboxy groups in the polycarboxylic acid.

In the polyester polyurethane (including the polyester-polyether polyurethane) in the pressure-sensitive adhesive layer 22, a second ratio is preferably 0.5 to 1.0, where the second ratio is the ratio of the total number of isocyanate groups in the multifunctional isocyanate (namely, the total number of isocyanate groups in the multifunctional isocyanate to constitute multifunctional isocyanate-derived units) to the total number of carboxy groups in the polycarboxylic acid (namely, the total number of carboxy groups in the polycarboxylic acid to constitute polycarboxylic acid-derived units). This configuration is advantageous for allowing the pressure-sensitive adhesive layer 22 in the identification sheet X1 to actually have both high transparency and good cohesiveness.

In the polyester-polyether polyurethane, when contained in the pressure-sensitive adhesive layer 22, a third ratio is preferably 1.5 to 9.0, where the third ratio is the ratio of the total number of hydroxy groups in the first polyhydric alcohol (namely, the total number of hydroxy groups in the first polyhydric alcohol to constitute first-polyhydric alcohol-derived units) to the total number of hydroxy groups in the second polyhydric alcohol (namely, the total number of hydroxy groups in the second polyhydric alcohol to constitute second-polyhydric alcohol-derived units). This configuration is advantageous for allowing the pressure-sensitive adhesive layer 22 in the identification sheet X1 to surely have high thermal stability, low fluidity, and high transparency and to still actually have both good cohesiveness and good flexibility.

Assume that the second polyhydric alcohol to constitute the polyester-polyether polyurethane in the pressure-sensitive adhesive layer 22 is a polypropylene glycol, a polyethylene glycol, or another multimer (polymer) derived from a single type of monomer. In this case, in the polyester-polyether polyurethane, a fourth ratio is preferably 0.1 to 0.4, and more preferably 0.12 to 0.34, where the fourth ratio is the ratio of the total number of hydroxy groups in the first polyhydric alcohol (namely, the total number of hydroxy groups in the first polyhydric alcohol to constitute first-polyhydric alcohol-derived units) to the total number of units derived from the single type of monomer in the second polyhydric alcohol, which is the multimer. The fourth ratio, which relates to the first and second polyhydric alcohols to form the polyester-polyether polyurethane, is preferably 0.1 to 0.4, and more preferably 0.12 to 0.34, for balancing between the technical effects mentioned relating to the first polyhydric alcohol and the technical effects mentioned relating to the second polyhydric alcohol. In addition, the fourth ratio is preferably 0.1 or more for restraining excessive hydrophilicity of the polyester-polyether polyurethane in the pressure-sensitive adhesive layer 22 and, consequently, for restraining excessive hydrophilicity of the pressure-sensitive adhesive layer 22, and advantageous for allowing the adhesive face 22a in the identification sheet X1 to surely have collectability of a hydrophobic component (such as an oil component in a secretion that forms fingerprints).

The first ratio, the second ratio, the third ratio, and the fourth ratio are values each of which may be determined on the basis of the abundance ratios or relative numbers of the various substructures or functional groups in the polymer. The abundance ratio or relative number of each of various substructures or functional groups in a polymer can be controlled by adjusting relative amounts or proportions of various material monomers used for the synthesis of the polymer. The abundance ratio or relative number of each of various substructures or functional groups in the resulting polymer may be determined typically through a process as follows.

Initially, the polymer to be analyzed (analyte polymer) is purified. For example, a material containing the analyte polymer and constituting the pressure-sensitive adhesive layer is stirred in a predetermined solvent to give a crude polymer solution. After removing solvent-insoluble matter from the solution by filtration, a solvent-soluble, low molecular weight non-polymer component is removed from the solution by gel permeation chromatography (GPC) to give a polymer-containing solution, from which the solvent is distilled off. The solvent is selected or determined depending on properties of the polymer, such as solubility. The solvent may be selected typically from dimethylformamide, chloroform, methylene chloride, tetrahydrofuran, acetone, dimethyl sulfoxide, methanol, ethanol, toluene, and water. Next, a sample solution is prepared by dissolving the analyte polymer after the purification in a predetermined deuterated solvent for NMR measurement. Alternatively, a sample solution may be prepared by subjecting the analyte polymer to a hydrolysis treatment for the hydrolysis of ester bonds and urethane bonds in the analyte polymer to give various fragments or monomers derived from the analyte polymer, and dissolving the fragments or monomers in a deuterated solvent. Further alternatively, a sample solution may be prepared by subjecting the analyte polymer to a solvolysis treatment using a deuterated solvent that can solvolyze ester bonds and urethane bonds in the analyte polymer to give a deuterated solvent containing various fragments or monomers derived from the analyte polymer, and preparing the sample solution from the resulting deuterated solvent. The various fragments or monomers resulting from the hydrolysis treatment or solvolysis treatment and being derived from the analyte polymer may be fractionated into fractions by GPC, and sample solutions for NMR measurement may be individually prepared on the fraction-to-fraction basis. The deuterated solvent is selected or determined depending on the properties of the polymer, such as solubility. The deuterated solvent for use herein may be selected typically from deuterated chloroform, deuterated methylene chloride, deuterated tetrahydrofuran, deuterated acetone, deuterated dimethyl sulfoxide, deuterated N,N-dimethylformamide, deuterated methanol, deuterated ethanol, and heavy water. Next, the sample solution is subjected to ¹H-NMR measurement to give a ¹H-NMR spectrum. The resulting spectrum is subjected to waveform separation, and intensity ratios (signal integral ratios) of signals assigned to various substructures or functional groups are determined. Then, on the basis of the signal intensity ratios, the abundance ratios or relative numbers of the various substructures or functional groups in the polymer are determined. The abundance ratios or relative numbers of various substructures or functional groups in the analyte polymer can be determined typically by the above procedure.

The pressure-sensitive adhesive layer 22 may contain an antioxidant and/or a so-called photostabilizer, in addition to the pressure-sensitive adhesive.

The identification sheet X1 having the configurations as above may be produced typically by the following procedure. Initially, the backing sheet 10 and the pressure-sensitive adhesive sheet 20 are individually prepared. The backing sheet 10 can be prepared by forming and laminating the writable layer 12 onto the base layer 11, and forming and laminating the background layer 13 and the release layer 14 onto the base layer 11. The writable layer 12 and the background layer 13 may be formed typically by a printing technique. The release layer 14 may be formed typically by a technique such as dry lamination or extrusion lamination. The pressure-sensitive adhesive sheet 20 may be formed typically by applying a pressure-sensitive adhesive composition onto the substrate layer 21 and drying the applied pressure-sensitive adhesive composition. The identification sheet X1 can be produced typically by the above procedure.

Using the identification sheet X1, a target material may be collected typically by the following procedure. Initially, the pressure-sensitive adhesive sheet 20 is removed from the mount face 10b of the backing sheet 10, and the target material is then attached to the exposed pressure-sensitive adhesive layer 22 or the adhesive face 22a of the pressure-sensitive adhesive sheet 20. Non-limiting examples of the target material include fingerprints, footprints, tire marks, and hairs. Assume that, among fingerprints left typically at a crime scene, a latent fingerprint is to be collected as the target material, where the latent fingerprint is invisible to the naked eye. In this case, for example, a predetermined surface or region where the presence of the latent fingerprint is estimated is subjected to a treatment using a fine powder such as a fine aluminum powder. When the latent fingerprint is present in the region, the treatment allows the fine powder to be attached to the latent fingerprint to thereby visualize the pattern shape (including ridges) of the fingerprint. The adhesive face 22a of the pressure-sensitive adhesive sheet 20, which has been removed from the backing sheet 10, is pressed to, and then removed from, the region. Thus, the visualized fingerprint is transferred onto the adhesive face 22a of the pressure-sensitive adhesive sheet 20. The collection of such a fingerprint may be performed by a technique using an Egyptian blue pigment (EB), in which the latent fingerprint is collected at the adhesive face 22a of the pressure-sensitive adhesive sheet 20, and is then acted upon by a predetermined treatment liquid to color and visualize (develop) the pattern shape of the fingerprint. The resulting pressure-sensitive adhesive sheet 20 to which the target material such as a fingerprint is attached typically by the above procedure is laminated again onto the mount face 10b of the original backing sheet 10. In the lamination, the adhesive face 22a bearing the target material is attached onto the mount face 10b. In the preprinted box at the outer face 10a of the backing sheet 10, data or information relating to the collection of the target material is written or recorded, and a collector and a witness individually sign and seal therein, where the data include date, collection number, case name, collection place, and collected material name (item name).

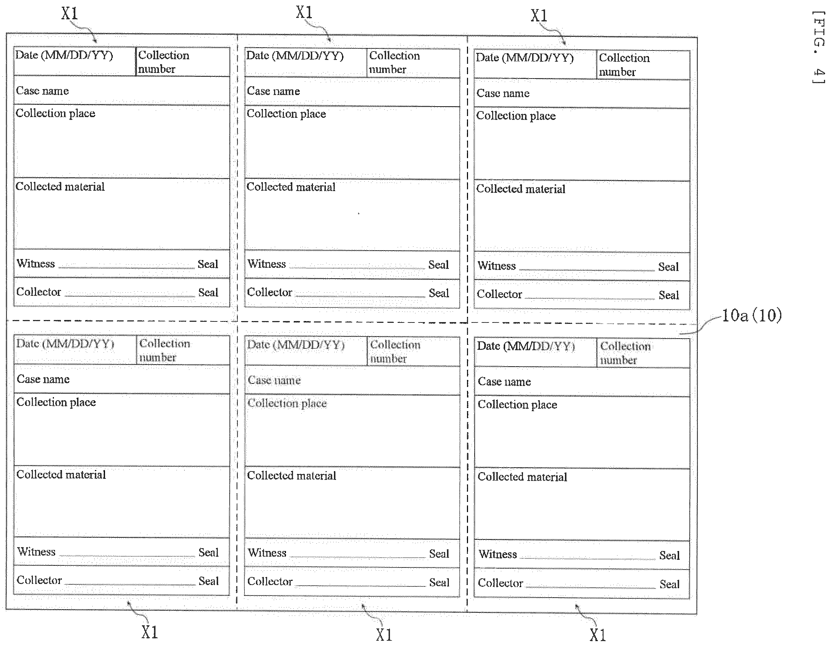

In possible configurations of the identification sheet X1, two or more identification sheets X1 are arrayed and unified, as illustrated in FIG. 4. FIG. 4 illustrates a configuration in which six identification sheets X1 are arrayed and unified. The identification sheet including identification sheets X1 being arrayed and unified may be cut into pieces having a necessary size before use. In a possible configuration of the identification sheet, two or more identification sheets are arrayed and unified; and this is also applicable to identification sheets relating to modifications as described below.

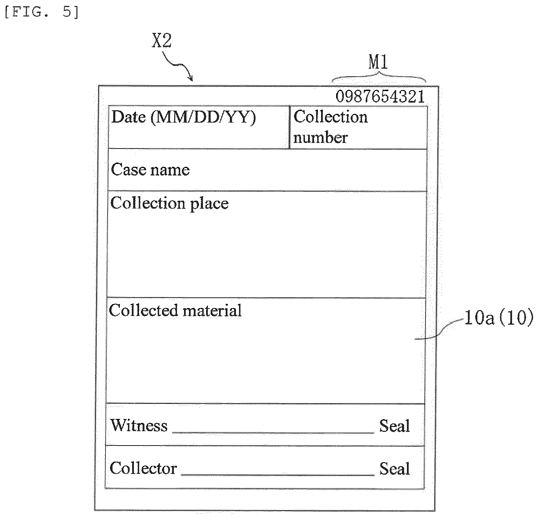

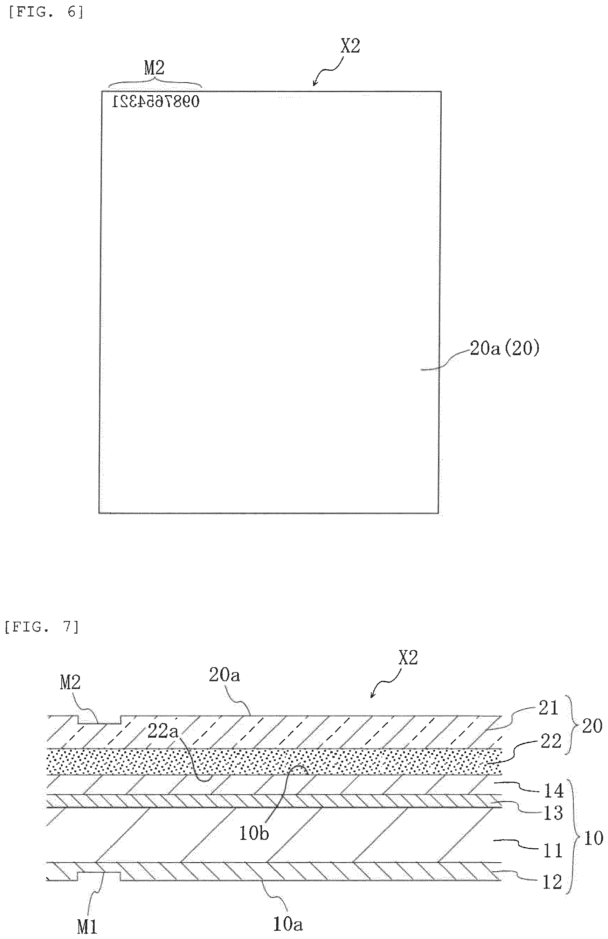

FIGs. 5 to 7 illustrate an identification sheet X2 according to one modification of the identification sheet X1. FIGs. 5 to 7 are a plan view, another plan view, and a partial enlarged cross-sectional view, respectively, of the identification sheet X2.

The identification sheet X2 includes a backing sheet 10 and a pressure-sensitive adhesive sheet 20, which are separable (removable) from each other. The backing sheet 10 has a multilayer structure including a base layer 11, a writable layer 12, a background layer 13, and a release layer 14. The pressure-sensitive adhesive sheet 20 has a multilayer structure including a substrate layer 21 and a pressure-sensitive adhesive layer 22. The identification sheet X2 has an outer face 10a and an outer face 20a, where the outer face 10a can serve as an outer surface defined by the backing sheet 10, and the outer face 20a can serve as an outer surface defined by the pressure-sensitive adhesive sheet 20. The outer face 10a bears a distinguishing mark M1, which carries distinguishing information. The outer face 10a bearing the distinguishing mark M1 may be covered with a transparent protective film. The outer face 20a bears a distinguishing mark M2, which carries distinguishing information. The outer face 20a bearing the distinguishing mark M2 may be covered with a transparent protective film. The identification sheet X2 as above is different from the identification sheet X1 in having the distinguishing mark M1 at the outer face 10a defined by the backing sheet 10; and in having the distinguishing mark M2 at the outer face 20a defined by the pressure-sensitive adhesive sheet 20. The identification sheet X2 has configurations similar to those described above relating to the identification sheet X1, except for configurations relating to the distinguishing marks M1 and M2. The identification sheet X2 includes the pressure-sensitive adhesive layer 22 suitable to resist thermal deformation, as with the identification sheet X1.

The distinguishing marks M1 and M2 are independently engraved marks. As used herein, the term "engraved mark" refers to a mark formed by an engraving technique, which gives a concave shape in a face to be marked. Non-limiting examples of the engraving technique include laser marking and air pen marking. The laser marking may be performed typically using any of CO₂ laser, UV laser, green laser, and fiber laser. The air pen marking is a marking technique that uses dot pressurization with a superhard stylus vibrating at high speed.

These distinguishing marks M1 and M2 are designed to correspond to each other, so as to function as a pair of cross-check information to determine whether a backing sheet 10 with the distinguishing mark M1 and a pressure-sensitive adhesive sheet 20 with the distinguishing mark M2 correspond to each other. Specifically, in an embodiment, the distinguishing marks M1 and M2 have pattern shapes identical to each other and function as a pair of cross-check information. In another embodiment, the distinguishing marks M1 and M2 have pattern shapes different from each other and function as a pair of cross-check information. In still another embodiment, the identification sheet X2 is designed so as to have a partial region that is transparent all over the thickness direction of the sheet X2. In this embodiment, the distinguishing mark M1 is disposed in the partial region in the outer face 10a; the distinguishing mark M2 is disposed in the partial region in the outer face 20a; and these distinguishing marks M1 and M2 can be seen at one time from the outer face 10a side or from the outer face 20a side and function as a pair of cross-check information. Non-limiting examples of elementary shapes to be included in the pattern shapes of the distinguishing marks M1 and M2 include numerical characters, alphabetic characters, hiragana characters, katakana characters, kanji characters, symbols, bar codes, QR Codes (quick response codes), and other two-dimensional codes. Examples of the bar codes include one-dimensional bar codes and two-dimensional bar codes. The distinguishing marks M1 and M2 may each independently have a pattern shape including elementary shapes of the same kind, or a patter shape including elementary shapes of different kinds. FIGs. 5 to 7 illustrate an embodiment in which the distinguishing marks M1 and M2 are both marks resulting from laser marking, have pattern shapes being identical to each other and including a sequence of numerical characters, and function as a pair of cross-check information.

The identification sheet X2 having the configurations as above may be produced typically by the following procedure. Initially, the backing sheet 10 and the pressure-sensitive adhesive sheet 20 are prepared independently. Next, the mount face 10b in the backing sheet 10 is laminated onto the adhesive face 22a in the pressure-sensitive adhesive sheet 20. Next, the distinguishing mark M1 is formed at the outer face 10a defined by the backing sheet 10, and, separately, the distinguishing mark M2 is formed at the outer face 20a defined by the pressure-sensitive adhesive sheet 20. Non-limiting examples of the marking technique include laser marking and air pen marking. The laser marking may be performed typically using any of CO₂ laser, UV laser, green laser, and fiber laser. The air pen marking is a marking technique that uses dot pressurization with a superhard stylus vibrating at high speed. After the distinguishing mark M1 is formed, a transparent protective film may be formed to cover the outer face 10a of the backing sheet 10. Likewise, after the distinguishing mark M2 is formed, a transparent protective film may be formed to cover the outer face 20a of the pressure-sensitive adhesive sheet 20. The identification sheet X2 can be produced typically by the above procedure.

High heat resistance may be required of the writable layer 12 of the backing sheet 10, as described above. Assume that laser marking is employed to form the distinguishing mark M1 at the outer face 10a defined by the writable layer 12 of the backing sheet 10. In this case, the higher the heat resistance of the constituent material of the writable layer is, the more preferred from the viewpoint of restraining collapse typically of drawn lines due to excessive melting of the constituent material of the writable layer upon laser marking.

The higher the transparency of the substrate layer 21 is, the more preferred from the viewpoint of surely providing visibility of the target material, when held between the backing sheet 10 and the pressure-sensitive adhesive sheet 20 and seen from the pressure-sensitive adhesive sheet 20 side, as described above. Assume that laser marking is employed to form the distinguishing mark M2 at the outer face 20a defined by the substrate layer 21 of the pressure-sensitive adhesive sheet 20. In this case, for example, the drawn lines tend to more resist coloring upon laser marking, with increasing transparency of the substrate layer 21.

The higher the transparency of the pressure-sensitive adhesive layer 22 is, the more preferred from the viewpoint of surely providing the visibility of the target material, when held between the backing sheet 10 and the pressure-sensitive adhesive sheet 20 and seen from the pressure-sensitive adhesive sheet 20, as described above. Assume that laser marking is employed to form at least one of the distinguishing mark M1 and the distinguishing mark M2. In this case, the high transparency of the pressure-sensitive adhesive layer 22 contributes to restraining temperature rise in the pressure-sensitive adhesive layer 22 due to laser beam absorption, and to eliminating or minimizing thermal deformation of the pressure-sensitive adhesive layer 22.

Using the identification sheet X2, a target material may be collected typically by the following procedure. The pressure-sensitive adhesive sheet 20 with the distinguishing mark M2 at the outer face 20a is removed from the mount face 10b of the backing sheet 10 bearing the distinguishing mark M1 at the outer face 10a. The target material is then attached to the exposed pressure-sensitive adhesive layer 22 or the adhesive face 22a of the pressure-sensitive adhesive sheet 20. Non-limiting examples of the target material include fingerprints, footprints, tire marks, and hairs. The identification sheet X2 enables collection of a latent fingerprint as the target material typically by the technique using a powder or the technique using the EB, by a procedure similar to the procedure of using the identification sheet X1 described above. The pressure-sensitive adhesive sheet 20 with the distinguishing mark M2, to which the target material such as a fingerprint is attached, is then laminated again onto the mount face 10b of the original backing sheet 10 with the distinguishing mark M1. In the lamination, the adhesive face 22a bearing the target material is attached onto the mount face 10b. In the preprinted box at the outer face 10a of the backing sheet 10, data or information relating to the collection of the target material is written or recorded, and the collector and the witness individually sign and seal therein, where the data or information includes date, collection number, case name, collection place, and collected material name (item name).

In the identification sheet X2, the distinguishing mark M1 at the backing sheet 10 and the distinguishing mark M2 at the pressure-sensitive adhesive sheet 20, where the two marks correspond to each other, form a pair in the single identification sheet. This certifies that the backing sheet 10 and the pressure-sensitive adhesive sheet 20 correspond to each other. Assume that, in the identification sheet X2 after the collection of the target material, the pressure-sensitive adhesive sheet 20, which bears the true target material and adheres to the backing sheet 10, is removed and replaced with another pressure-sensitive adhesive sheet, where the other pressure-sensitive adhesive sheet is a pressure-sensitive adhesive sheet that lacks the distinguishing mark M2 corresponding to the distinguishing mark M1 of the backing sheet 10. In this case, there can be determined the absence of mutual correspondence between the other pressure-sensitive adhesive sheet after replacement and the backing sheet 10. This is because the other pressure-sensitive adhesive sheet lacks the distinguishing mark M2 corresponding to the distinguishing mark M1 of the backing sheet 10. Assume that, in the identification sheet X2 after the collection of the target material, the backing sheet, to which the pressure-sensitive adhesive sheet 20 bearing the true target material adheres and on which data such as various information for the target material are recorded, is removed and replaced with another backing sheet, where the other backing sheet is a backing sheet that lacks the distinguishing mark M1 corresponding to the distinguishing mark M2 of the pressure-sensitive adhesive sheet 20. In this case, there can be determined the absence of mutual correspondence between the other backing sheet after replacement and the pressure-sensitive adhesive sheet 20. This is because the other backing sheet lacks the distinguishing mark M1 corresponding to the distinguishing mark M2 of the pressure-sensitive adhesive sheet 20. Namely, if the identification sheet X2 undergoes such a replacement as above, the replacement can be detected or recognized. This is because the identification sheet X2 includes the backing sheet 10 and the pressure-sensitive adhesive sheet 20, where the backing sheet 10 bears the distinguishing mark M1, which is one of a pair of cross-check information; and the pressure-sensitive adhesive sheet 20 bears the distinguishing mark M2, which is the other of the pair of cross-check information. The identification sheet X2 as above is suitable for restraining a tampering act on the identification sheet after the collection of the target material and for restraining the mistaking of the pressure-sensitive adhesive sheet 20 or the backing sheet 10 for another one during a collecting process and an identifying operation for the target material. Consequently, the identification sheet X2 is suitable for surely providing the admissibility of evidence required of the target material collected typically in a criminal case.

In addition, in the identification sheet X2, the distinguishing mark M1 has been formed at the outer face 10a of the backing sheet 10, separately from the distinguishing mark M2; and the distinguishing mark M2 has been formed at the outer face 20a of the pressure-sensitive adhesive sheet 20, separately from the distinguishing mark M1. The distinguishing mark M1 in the identification sheet X2 is neither one formed by copying as a result of a transferring action of a coloring component constituting part of another distinguishing mark; nor one formed by copying as a result of a physical and/or chemical reaction caused typically by local heating, pressurization, or photoirradiation in the formation of another distinguishing mark. The distinguishing mark M2 in the identification sheet X2 is neither one formed by copying as a result of a transferring action of a coloring component constituting part of another distinguishing mark; nor one formed by copying as a result of a physical and/or chemical reaction caused typically by local heating, pressurization, or photoirradiation in the formation of another distinguishing mark. The distinguishing marks M1 and M2 are engraved marks individually or separately formed or engraved in the identification sheet X2. This configuration is advantageous for restraining or avoiding the deterioration of the distinguishing marks M1 and M2. Accordingly, the configuration is advantageous for allowing the distinguishing marks M1 and M2 to keep on functioning as a pair of cross-check information so as to determine the mutual correspondence between a backing sheet 10 with the distinguishing mark M1 and a pressure-sensitive adhesive sheet 20 with the distinguishing mark M2.

As described above, the identification sheet X2 includes the pressure-sensitive adhesive layer 22, which adequately resists thermal deformation, as with the identification sheet X1; and, in addition, the identification sheet X2 is suitable for restraining the tampering act and mistaking relating to the identification sheet, and, consequently, is suitable for keeping on surely providing admissibility of evidence in the collected target material, where the admissibility of evidence is required typically in a criminal case.

The configuration, where the distinguishing marks M1 and M2 in the identification sheet X2 are engraved marks, is advantageous for allowing faces to be marked (outer faces 10a and 20a) to less undergo a shape change (such as formation of scratch marks) other than the formed distinguishing marks M1 and M2. For example, scratch marks, if present in the outer face 20a defined by the transparent pressure-sensitive adhesive sheet 20, may impede the operation to identify the target material (such as fingerprints) collected by the identification sheet X2.

In addition, when laser marking is employed to form at least one of the distinguishing mark M1 and the distinguishing mark M2, the pattern shape of the distinguishing mark to be formed can be designed or determined with high degree of freedom. This contributes to higher productivity of the identification sheet.

The configuration, where the identification sheet X2 bears or includes an engraved mark formed by laser marking as at least one of the distinguishing mark M1 and the distinguishing mark M2, is also advantageous for surely providing removability or releasability between the backing sheet 10 and the pressure-sensitive adhesive sheet 20. Assume that, in an identification sheet including a backing sheet and a pressure-sensitive adhesive sheet laminated on each other, a laser mark is formed as a through hole penetrating the backing sheet by laser irradiation from the backing sheet side, or a laser mark is formed as a through hole penetrating the pressure-sensitive adhesive sheet by laser irradiation from the pressure-sensitive adhesive sheet side, or a laser mark is formed as a through hole penetrating the entire identification sheet by laser irradiation. In these cases, the pressure-sensitive adhesive is softened and melted at or around an area, in which the through hole is formed, in a surface of the pressure-sensitive adhesive layer of the pressure-sensitive adhesive sheet, where the surface faces the backing sheet. Thus, the pressure-sensitive adhesive layer is liable to fuse to the backing sheet in the area. The fusion, if occurring, may impede the separation, namely, the removal between the backing sheet and the pressure-sensitive adhesive sheet, and/or may cause the pressure-sensitive adhesive layer to be significantly damaged, because the pressure-sensitive adhesive layer at the fused portion is pulled by the backing sheet upon removal. The configuration, where the identification sheet X2 bears or includes an engraved mark formed by laser marking as at least one of the distinguishing mark M1 and the distinguishing mark M2, is advantageous for avoiding or minimizing the occurrence of local fusion between the backing sheet 10 and the pressure-sensitive adhesive sheet 20 and for surely providing removability between the two sheets. Softening and melting of the pressure-sensitive adhesive in the adhesive face causes deformation of the softened-melted area in the pressure-sensitive adhesive layer. The resulting deformation or unevenness in the adhesive face may lower the adhesion between the backing sheet and the pressure-sensitive adhesive sheet when the two sheets after removal from each other are laminated again on each other. The configuration, where the identification sheet X2 bears or includes an engraved mark formed by laser marking as at least one of the distinguishing mark M1 and the distinguishing mark M2, is advantageous for avoiding or minimizing the deformation or unevenness at the adhesive face 22a in the pressure-sensitive adhesive sheet 20 and for surely providing adhesion between the backing sheet 10 and the pressure-sensitive adhesive sheet 20 after the two sheets are laminated again on each other.

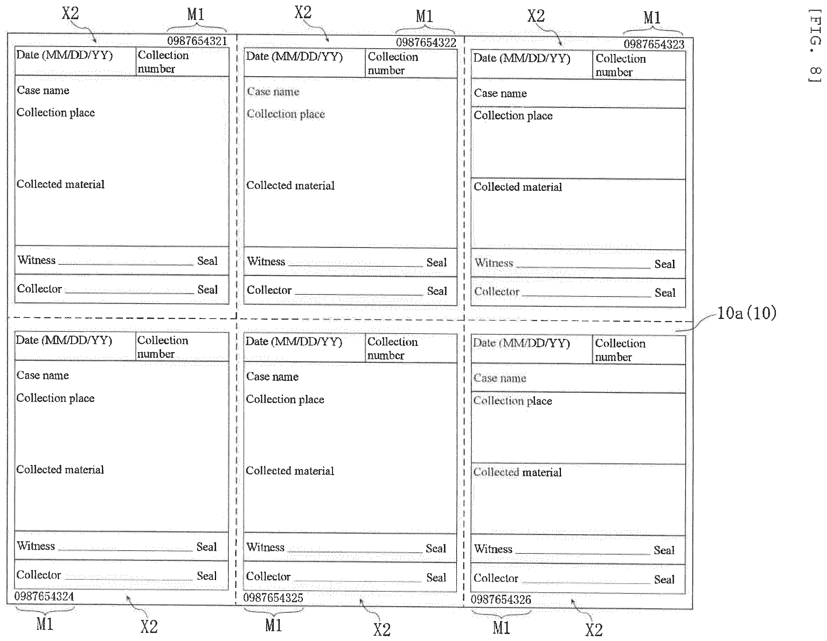

In possible configurations of the identification sheet X2 as above, two or more identification sheets X2 may be arrayed and unified. FIG. 8 illustrates a configuration in which six identification sheets X2 are arrayed and unified. In the configuration as above, distinguishing marks M1 are made individually in boxes at the outer face 10a. The distinguishing marks M1 have pattern shapes different from one another. At the outer face 20a (not shown), distinguishing marks M2 (not shown) are made respectively corresponding to the distinguishing marks M1 at the outer face 10a. The distinguishing marks M2 at the outer face 20a also have pattern shapes different from one another. The pairs of distinguishing marks M1 and M2, which function as pairs of cross-check information, are present in individual boxes. The identification sheet including identification sheets X2 being arrayed and unified may be cut into pieces having a necessary size before use. As described above, in a possible configuration of the identification sheet X2, two or more identification sheets X2 are arrayed and unified; and this is also applicable to each of identification sheets according to modifications described below.

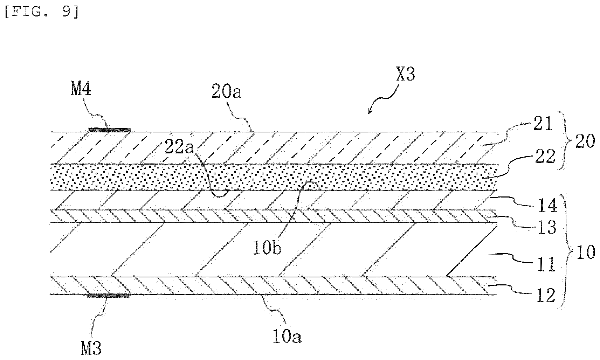

FIG. 9 is a partial enlarged cross-sectional view of an identification sheet X3 according to one modification of the identification sheet X1. The identification sheet X3 includes a backing sheet 10 and a pressure-sensitive adhesive sheet 20, which are separable (removable) from each other. The backing sheet 10 has a multilayer structure including a base layer 11, a writable layer 12, a background layer 13, and a release layer 14. The pressure-sensitive adhesive sheet 20 has a multilayer structure including a substrate layer 21 and a pressure-sensitive adhesive layer 22. The identification sheet X3 has an outer face 10a and an outer face 20a. The outer face 10a can serve as an outer surface defined by the backing sheet 10. The outer face 20a can serve as an outer surface defined by the pressure-sensitive adhesive sheet 20. The outer face 10a bears a distinguishing mark M3 carrying distinguishing information. The outer face 20a bears a distinguishing mark M4 carrying distinguishing information. The identification sheet X3 as above is different from the identification sheet X1 in bearing the distinguishing mark M3 at the outer face 10a defined by the backing sheet 10 and in bearing the distinguishing mark M4 at the outer face 20a defined by the pressure-sensitive adhesive sheet 20. The identification sheet X3 has configurations similar to those described above relating to the identification sheet X1, except for configurations relating to the distinguishing marks M3 and M4. As with the identification sheet X1, the identification sheet X3 includes the pressure-sensitive adhesive layer 22, which adequately resists thermal deformation.

The distinguishing marks M3 and M4 are independently printed marks. As used herein, the term "printed mark" refers to a mark formed by a printing technique on a face to be marked. Non-limiting examples of the printing technique include inkjet printing, thermal transfer printing, gravure printing, relief printing, silk-screen printing, and stamp printing. The distinguishing marks M3 and M4 may each independently be formed by printing with an ink having a large contrast difference from a corresponding background color, or with an ink having a small contrast difference from the corresponding background color. Alternatively, the distinguishing marks M3 and M4 may each independently be formed by printing typically with an ink that develops a color upon ultraviolet irradiation.

These distinguishing marks M3 and M4 are designed to correspond to each other, so as to function as a pair of cross-check information for determining whether a backing sheet 10 with the distinguishing mark M3 and a pressure-sensitive adhesive sheet 20 with the distinguishing mark M4 correspond to each other. Specifically, the distinguishing marks M3 and M4 may have pattern shapes identical to each other and function as a pair of cross-check information; or may have pattern shapes different from each other and function as a pair of cross-check information. In another embodiment, the identification sheet X3 is designed to have a partial region that is transparent all over the thickness direction of the sheet X3. In this embodiment, the distinguishing mark M3 is disposed in the partial region in the outer face 10a; the distinguishing mark M4 is disposed in the partial region in the outer face 20a; and these distinguishing marks M3 and M4 can be seen at one time from the outer face 10a side or from the outer face 20a side and function as a pair of cross-check information. Elementary shapes which may be included in the pattern shapes of the distinguishing marks M3 and M4 are, for example, as with the elementary shapes which may be included in the pattern shapes of the distinguishing marks M1 and M2 in the identification sheet X2.

The identification sheet X3, which has the configurations as above, may be produced typically by a procedure as with the production process for the identification sheet X2, except for forming printed marks, instead of the engraved marks, at the outer faces 10a and 20a. The identification sheet X3 may be used by a procedure similar to that for the identification sheet X1 as described above.

In the identification sheet X3, the distinguishing mark M3 at the backing sheet 10 and the distinguishing mark M4 at the pressure-sensitive adhesive sheet 20, where the two marks correspond to each other, form a pair in the single identification sheet. This certifies that the backing sheet 10 and the pressure-sensitive adhesive sheet 20 correspond to each other. This is advantageous for restraining a tampering act and mistaking of the sheet(s) for another one in the identification sheet, as with that the pairing (correspondence) of the distinguishing marks M1 and M2 in the identification sheet X2 certifies that the backing sheet 10 and the pressure-sensitive adhesive sheet 20 correspond to each other. In the identification sheet X3, the distinguishing mark M3 has been formed at the outer face 10a of the backing sheet 10, separately from the distinguishing mark M4; and the distinguishing mark M4 has been formed at the outer face 20a of the pressure-sensitive adhesive sheet 20, separately from the distinguishing mark M3. The distinguishing marks M3 and M4 are each neither one formed by copying as a result of a transferring action of a coloring component constituting part of another distinguishing mark; nor one formed by copying as a result of a physical and/or chemical reaction caused typically by local heating, pressurization, or photoirradiation in the formation of another distinguishing mark. This configuration is advantageous for restraining or avoiding the deterioration of the distinguishing marks M3 and M4. The configuration is therefore advantageous for allowing the distinguishing marks M3 and M4 to keep on functioning as a pair of cross-check information to determine whether a backing sheet 10 with the distinguishing mark M3 and a pressure-sensitive adhesive sheet 20 with the distinguishing mark M4 correspond to each other.

As described above, the identification sheet X3 includes the pressure-sensitive adhesive layer 22, which adequately resists thermal deformation, as with the identification sheet X1. In addition, the identification sheet X3 is suitable for restraining the tampering act and mistaking relating to the identification sheet and, consequently, is suitable for keeping on surely providing admissibility of evidence of the collected target material, where the admissibility of evidence is required typically in a criminal case.

In the identification sheet X3, the distinguishing mark M1, which is described above relating to the identification sheet X2, may be disposed at the outer face 10a defined by the backing sheet 10, instead of the distinguishing mark M3. Also in the identification sheet X3, the distinguishing mark M2, which is described above relating to the identification sheet X2, may be disposed at the outer face 20a defined by the pressure-sensitive adhesive sheet 20, instead of the distinguishing mark M4.

### Examples

### Example 1

### Preparation of Pressure-Sensitive Adhesive Solution

Material components were placed in a flask equipped with a reflux condenser, a nitrogen gas inlet tube, a stirrer, and a thermometer. The components were 0.089 mol of adipic acid (supplied by Wako Pure Chemical Industries, Ltd.), 0.117 mol of neopentyl glycol (supplied by Wako Pure Chemical Industries, Ltd.), and, as a solvent, 100 g of decahydronaphthalene (supplied by Wako Pure Chemical Industries, Ltd.). With introduction of nitrogen gas into the flask, the components in the flask were stirred at a temperature maintained in the range of 180°C to 200°C for 12 hours (dehydrative condensation). Next, after distilling off the solvent from the reaction solution in the flask, the residue was combined with tetrahydrofuran as a solvent. Next, after changing the temperature to 90°C, the solution in the flask was combined with 0.064 mol of hexamethylene diisocyanate (supplied by Wako Pure Chemical Industries, Ltd.), and 0.001 mol of dibutyltin dilaurate (supplied by Wako Pure Chemical Industries, Ltd.) as a urethane synthesis catalyst. The resulting solution in the flask was stirred for 15 hours with introduction of nitrogen gas into the flask (polyurethanization). Next, the solvent was distilled off from the reaction solution to leave a solid, the solid was combined with toluene, and yielded a pressure-sensitive adhesive solution (pressure-sensitive adhesive solution S1) containing a polyester polyurethane, which serves as a pressure-sensitive adhesive. The pressure-sensitive adhesive solution S1 had a solids concentration of 55 mass percent.

### Preparation of Transparent Pressure-Sensitive Adhesive Sheet

The pressure-sensitive adhesive solution S1 was applied onto a 75-µm thick transparent poly(ethylene terephthalate) (PET) film LUMIRROR T60 (trade name, supplied by Toray Industries Inc.) to constitute a substrate layer of a transparent pressure-sensitive adhesive sheet for use in an identification sheet. The applied solution was dried to form a 290-µm thick transparent pressure-sensitive adhesive layer (with area in plan view of 168 cm²). Thus, a required number of transparent pressure-sensitive adhesive sheets for use in an identification sheet according to Example 1 was prepared by the procedure as above.

### Example 2

### Preparation of Pressure-Sensitive Adhesive Solution

Material components were placed in a flask equipped with a reflux condenser, a nitrogen gas inlet tube, a stirrer, and a thermometer. The components were 0.096 mol of adipic acid (supplied by Wako Pure Chemical Industries, Ltd.), 0.127 mol of neopentyl glycol (supplied by Wako Pure Chemical Industries, Ltd.), 0.071 mol of a polypropylene glycol having a number average molecular weight (Mn) of 1000 (supplied by Wako Pure Chemical Industries, Ltd.), and, as a solvent, 100 g of decahydronaphthalene (supplied by Wako Pure Chemical Industries, Ltd.). The components in the flask were then stirred at a temperature maintained in the range of 180°C to 200°C for 12 hours with introduction of nitrogen gas into the flask (dehydrative condensation). Next, the solvent was distilled off from the reaction solution in the flask to leave a residue, and the residue was combined with tetrahydrofuran as a solvent. Next, after changing the temperature to 90°C, the solution in the flask was combined with 0.064 mol of hexamethylene diisocyanate (supplied by Wako Pure Chemical Industries, Ltd.), and 0.001 mol of dibutyltin dilaurate (supplied by Wako Pure Chemical Industries, Ltd.) as a urethane synthesis catalyst. The resulting solution in the flask was stirred for 15 hours with introduction of nitrogen gas into the flask (polyurethanization). Next, the solvent was distilled off from the reaction solution to leave a solid, the solid was combined with toluene, and yielded a pressure-sensitive adhesive solution (pressure-sensitive adhesive solution S2) containing a polyester-polyether polyurethane, which serves as a pressure-sensitive adhesive. The pressure-sensitive adhesive solution S2 had a solids concentration of 55 mass percent.

### Preparation of Transparent Pressure-Sensitive Adhesive Sheet

Except for using the pressure-sensitive adhesive solution S2 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Example 2 was prepared by the procedure as above.

### Example 3

A pressure-sensitive adhesive solution (pressure-sensitive adhesive solution S3) containing a polyester-polyether polyurethane, which serves as a pressure-sensitive adhesive, was prepared by a procedure similar to that in Example 2, except for using 0.035 mol of a polypropylene glycol having a number average molecular weight (Mn) of 2000 (supplied by Wako Pure Chemical Industries, Ltd.), instead of the polypropylene glycol having a number average molecular weight Mn of 1000, in the preparation of the pressure-sensitive adhesive solution. Except for using the pressure-sensitive adhesive solution S3 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Example 3 was prepared by the procedure as above.

### Example 4

A pressure-sensitive adhesive solution (pressure-sensitive adhesive solution S4) containing a polyester-polyether polyurethane, which serves as a pressure-sensitive adhesive, was prepared by a procedure similar to that in Example 2, except for using 0.024 mol of a polypropylene glycol having a number average molecular weight (Mn) of 3000 (supplied by Wako Pure Chemical Industries, Ltd.) instead of the polypropylene glycol having a number average molecular weight Mn of 1000 in the preparation of the pressure-sensitive adhesive solution. Except for using the pressure-sensitive adhesive solution S4 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Example 4 was prepared by the procedure as above.

### Example 5

A pressure-sensitive adhesive solution (pressure-sensitive adhesive solution S5) containing a polyester-polyether polyurethane, which serves as a pressure-sensitive adhesive, was prepared by a procedure similar to that in Example 2, except for, in the preparation of the pressure-sensitive adhesive solution, using adipic acid in an amount of 0.097 mol instead of 0.096 mol, using neopentyl glycol in an amount of 0.13 mol instead of 0.127 mol, and using 0.04 mol of a polypropylene glycol having a number average molecular weight Mn of 2000 (supplied by Wako Pure Chemical Industries, Ltd.) and 0.04 mol of a polypropylene glycol having a number average molecular weight Mn of 3000 (supplied by Wako Pure Chemical Industries, Ltd.) instead of the polypropylene glycol having a number average molecular weight Mn of 1000. Except for using the pressure-sensitive adhesive solution S5 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Example 5 was prepared by the procedure as above.

### Example 6

A pressure-sensitive adhesive solution (pressure-sensitive adhesive solution S6) containing a polyester-polyether polyurethane, which serves as a pressure-sensitive adhesive, was prepared by a procedure similar to that in Example 2, except for, in the preparation of the pressure-sensitive adhesive solution, using adipic acid in an amount of 0.07 mol instead of 0.096 mol, using neopentyl glycol in an amount of 0.105 mol instead of 0.127 mol, and using 0.028 mol of a polypropylene glycol having a number average molecular weight Mn of 2000 (supplied by Wako Pure Chemical Industries, Ltd.) instead of the polypropylene glycol having a number average molecular weight Mn of 1000. Except for using the pressure-sensitive adhesive solution S6 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Example 6 was prepared by the procedure as above.

### Comparative Example 1

Material components were placed in a flask equipped with a reflux condenser, a nitrogen gas inlet tube, a stirrer, and a thermometer. The components were 0.064 mol of hexamethylene diisocyanate (supplied by Wako Pure Chemical Industries, Ltd.), 0.064 mol of neopentyl glycol (supplied by Wako Pure Chemical Industries, Ltd.), 0.001 mol of dibutyltin dilaurate (supplied by Wako Pure Chemical Industries, Ltd.) as a urethane synthesis catalyst, and 100 g of decahydronaphthalene (supplied by Wako Pure Chemical Industries, Ltd.) as a solvent. The solution in the flask was stirred at 90°C for 15 hours with introduction of nitrogen gas into the flask. Next, the solvent was distilled off from the reaction solution to leave a solid, the solid was combined with toluene, and yielded a pressure-sensitive adhesive solution (pressure-sensitive adhesive solution S7) containing a polyurethane as a pressure-sensitive adhesive. The pressure-sensitive adhesive solution S7 had a solids concentration of 55 mass percent. Except for using the pressure-sensitive adhesive solution S7 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Comparative Example 1 was prepared by the procedure as above.

### Comparative Example 2

Material components were placed in a flask equipped with a reflux condenser, a nitrogen gas inlet tube, a stirrer, and a thermometer. The components were 0.064 mol of hexamethylene diisocyanate (supplied by Wako Pure Chemical Industries, Ltd.), 0.07 mol of a polypropylene glycol having a number average molecular weight Mn of 2000 (supplied by Wako Pure Chemical Industries, Ltd.), 0.001 mol of dibutyltin dilaurate (supplied by Wako Pure Chemical Industries, Ltd.) as a urethane synthesis catalyst, and 100 g of decahydronaphthalene (supplied by Wako Pure Chemical Industries, Ltd.) as a solvent. The solution in the flask was stirred at 90°C for 15 hours with introduction of nitrogen gas into the flask. Next, the solvent was distilled off from the reaction solution to leave a solid, the solid was combined with toluene, and yielded a pressure-sensitive adhesive solution (pressure-sensitive adhesive solution S8) containing a polyether polyurethane, which serves as a pressure-sensitive adhesive. The pressure-sensitive adhesive solution S8 had a solids concentration of 55 mass percent. Except for using the pressure-sensitive adhesive solution S8 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Comparative Example 2 was prepared by the procedure as above.

### Comparative Example 3

Material components were placed in a four-necked separable flask equipped with a water separator tube, a stirrer, and a thermometer. The components were 250 g (hydroxy group: 0.512 equivalent) of a polycarbonate diol PLACCEL CD210PL (trade name, having a hydroxy value of 115 KOH mg/g, supplied by Daicel Chemical Industries Ltd.), 51.8 g (acid group: 0.512 equivalent) of sebacic acid, and 127 mg of dibutyltin oxide as a catalyst. In the presence of a small amount of toluene as a solvent for discharging reaction water, the mixture was heated up to 180°C with the start of stirring, then held at 180°C, and thus the reaction was continued for about 24 hours. Next, the solvent was distilled off from the reaction solution to leave a solid, the solid was combined with toluene, and yielded a pressure-sensitive adhesive solution (pressure-sensitive adhesive solution S9) containing a polyester, which serves as a pressure-sensitive adhesive. The pressure-sensitive adhesive solution S9 had a solids concentration of 55 mass percent. Except for using the pressure-sensitive adhesive solution S9 instead of the pressure-sensitive adhesive solution S1, a 290-µm thick transparent pressure-sensitive adhesive layer was formed on a 75-µm thick transparent poly(ethylene terephthalate) film by a procedure similar to that in Example 1. Thus, a required number of transparent pressure-sensitive adhesive sheets for use in an identification sheet according to Comparative Example 3 was prepared by the procedure as above.

### Polymer Chemical Composition Analysis by NMR Measurement

The polymers contained as pressure-sensitive adhesives in the pressure-sensitive adhesive layers of the transparent pressure-sensitive adhesive sheets according to the examples and the comparative examples were analyzed for their chemical compositions. The analysis was performed by subjecting each of the polymers sequentially to purification, solvolysis treatment, and NMR measurement.

In the purification, initially, the pressure-sensitive adhesive layer was separated and sampled from a sample pressure-sensitive adhesive sheet. Next, the sampled pressure-sensitive adhesive layer was stirred in dimethylformamide (DMF) at 100°C for 48 hours and yielded a crude polymer solution. In the preparation of the crude polymer solution, 15 ml of DMF were added per 0.1 g of the pressure-sensitive adhesive layer. Next, solvent-insoluble matter was removed from the crude polymer solution by filtration. Next, solvent-soluble, low-molecular-weight non-polymer components were removed from the solution after the filtration treatment, by gel permeation chromatography (GPC) in specifications listed below. Next, the solvent was distilled off from a polymer-containing solution resulting from GPC.

### GPC Specifications

- GPC system: HLC-8120GPC (supplied by TOSOH CORPORATION)
- Columns: HXL Guard Column, TSKgel G4000HXL, TSKgel G5000HXL, and TSKgel GMHXL mixed-bed (all supplied by TOSOH CORPORATION) coupled in series
- Column temperature: 40°C
- Eluent: DMF
- Solvent flow rate: 1 ml/min
- Reference standard: polyethylene oxide
- Detector: differential refractometer RID-10A (supplied by Shimadzu Corporation)

The analyte polymer after the purification was subjected to the solvolysis treatment under a heating condition using deuterated methanol as a NMR measurement solvent. In the solvolysis treatment, deuterated methanol was used in excess (10 to 100 g) per gram of the analyte polymer. This gave a deuterated methanol solution containing various fragments or various monomers derived from the analyte polymer, and this solution was used as a sample solution for NMR measurement. The sample solution was subjected to ¹H-NMR measurement in specifications listed as follows and yielded a ¹H-NMR spectrum.

### Specifications of NMR Measurement

- NMR system: Bruker Biospin AVANCE III-600 with Cryo Probe
- Nuclide to be observed: ¹H
- Observation frequency: 600 MHz
- Observation temperature: 300K
- Chemical shift reference: 7.25 ppm
- Number of accumulation (measurement): 16

After waveform separation, intensity ratios (signal integral ratios) of signals assigned to various substructures or functional groups, which had been contained in the analyte polymer, were determined from the resulting spectrum. Using the signal intensity ratios and corresponding chemical formula weights such as molecular weights, there were determined, in the analyte polymer, a relative numerical content or mole ratio (H) of units derived from hexamethylene diisocyanate, a relative numerical content or mole ratio (A) of units derived from adipic acid, a relative numerical content or mole ratio (N) of units derived from neopentyl glycol, and a relative numerical content or mole ratio (P) of units derived from each polypropylene glycol. On the basis of these, a first ratio, namely (2N + 2P)/(2H + 2A), was determined, where the first ratio is the ratio of the sum of the total number (2N) of hydroxy groups assigned to the units derived from neopentyl glycol (first polyhydric alcohol) and the total number (2P) of hydroxy groups assigned to the units derived from polypropylene glycol (second polyhydric alcohol) to the sum of the total number (2H) of isocyanate groups assigned to the units derived from hexamethylene diisocyanate (multifunctional isocyanate) and the total number (2A) of carboxy groups assigned to the units derived from adipic acid (polycarboxylic acid). Likewise, a second ratio, namely (2H)/(2A), was determined, where the second ratio is the ratio of the total number (2H) of isocyanate groups assigned to the units derived from hexamethylene diisocyanate (multifunctional isocyanate) to the total number (2A) of carboxy groups assigned to the units derived from adipic acid (polycarboxylic acid). A third ratio, namely (2N)/(2P), was determined, where the third ratio is the ratio of the total number (2N) of hydroxy groups assigned to the units derived from neopentyl glycol (first polyhydric alcohol) to the total number (2P) of hydroxy groups assigned to the units derived from polypropylene glycol (second polyhydric alcohol). A fourth ratio, namely (2N)/(P units), was determined, where the fourth ratio is the ratio of the total number (2N) of hydroxy groups assigned to the units derived from neopentyl glycol (first polyhydric alcohol) to the total number (P units) of oxypropylene blocks as subunits contained in units derived from the polypropylene glycol (second polyhydric alcohol), where the propylene glycol is a polymer of oxypropylene. The results are given in Table 1.

### Laser Processability

How laser processing affects the pressure-sensitive adhesive layer was examined in each of the transparent pressure-sensitive adhesive sheets according to the examples and the comparative examples. Specifically, initially, laser marking was performed on a sample transparent pressure-sensitive adhesive sheet having a multilayer structure including the PET substrate layer and the pressure-sensitive adhesive layer. The laser marking was performed on the outer surface defined by the substrate layer (surface on opposite side to the pressure-sensitive adhesive layer) using a CO₂ laser marker LP-420 (trade name, supplied by Panasonic Industrial Devices SUNX Co., Ltd.). This laser marking gave a 100-mm long straight line as a laser mark on the substrate layer surface, at an energy output of the laser marker of 50% and a scanning speed of 500 mm/second. Next, five observation points were set at 20-mm regular intervals between the both ends of the straight laser mark, and cross sections at the observation points were cut out and each microscopically observed using a digital microscope (supplied by KEYENCE CORPORATION). In observation of the five observation points in the 100-mm long straight laser mark, a sample suffering from no gap (air space) between the substrate layer and the pressure-sensitive adhesive layer was evaluated as "very good (VG)", a sample suffering from air space at one or two points was evaluated as "good", a sample suffering from air space at three or four points was evaluated as "fair", and a sample suffering from air space at all the five points was evaluated as "poor". The results for Examples 1 to 5 and Comparative Examples 1 and 2 are given in Table 1. The sample according to Comparative Example 3 was evaluated as "fair". The transparent pressure-sensitive adhesive sheets according to Examples 2 to 6 were evaluated as "very good", where each of these pressure-sensitive adhesive sheets contained a polyester-polyether polyurethane as a pressure-sensitive adhesive in the pressure-sensitive adhesive layer. The transparent pressure-sensitive adhesive sheet according to Example 1 was evaluated as "good", where the pressure-sensitive adhesive sheet contained a polyester polyurethane as a pressure-sensitive adhesive in the pressure-sensitive adhesive layer. The transparent pressure-sensitive adhesive sheets according to Comparative Examples 1 to 3 were evaluated as "poor" or "fair", where these pressure-sensitive adhesive sheets each contained, in the pressure-sensitive adhesive layer, a pressure-sensitive adhesive that is neither a polyester polyurethane nor a polyester-polyether polyurethane.

### Measurement of Softening Temperature of Pressure-Sensitive Adhesive Layer

The transparent pressure-sensitive adhesive sheets according to the examples and the comparative examples were each evaluated for the softening temperature of the pressure-sensitive adhesive layer. Specifically, initially, a test piece (6 mm by 6 mm) was cut out from a sample transparent pressure-sensitive adhesive sheet having a multilayer structure of the PET substrate layer and the pressure-sensitive adhesive layer. Next, as a measurement object, the center of the test piece at the pressure-sensitive adhesive layer side was subjected to measurement of the softening temperature of the pressure-sensitive adhesive layer using a thermomechanical analyzer TMA/SS7100 (trade name, supplied by SII NanoTechnology Inc.) in a nitrogen stream at a flow rate of 200 ml/min. This measurement was performed in a penetration mode of the analyzer using a needle penetrator probe having a tip diameter of 1 mm under a load of 1470 mN, in which the tip of the probe was brought in contact with the pressure-sensitive adhesive layer. The measurement was performed under temperature conditions in which the temperature was raised from a start temperature of 150°C up to an end temperature of 320°C, at a rate of temperature rise of 10°C/min in the range from the start temperature to the end temperature. The pressure-sensitive adhesive layers according to Examples 2 to 4 each had a softening temperature of 243°C.

**[Table 1]**

| | | Example 1 | Example 2 | Example 3 | Example 4 | Example 5 | Example 6 | Comparative Example 1 | Comparative Example 2 |
|---|---|---|---|---|---|---|---|---|---|
| Compositional ratio in polymer | H | 0.064 | 0.064 | 0.064 | 0.064 | 0.064 | 0.064 | 0.064 | 0.064 |
| | A | 0.089 | 0.096 | 0.096 | 0.096 | 0.097 | 0.07 | 0 | 0 |
| | N | 0.117 | 0.127 | 0.127 | 0.127 | 0.13 | 0.105 | 0.064 | 0 |
| | P with Mn of 1000 | 0 | 0.071 | 0 | 0 | 0.04 | 0 | 0 | 0 |
| | P with Mn of 2000 | 0 | 0 | 0.035 | 0 | 0.04 | 0.028 | 0 | 0.07 |
| | P with Mn of 3000 | 0 | 0 | 0 | 0.024 | 0 | 0 | 0 | 0 |
| First ratio | (2N + 2P)/(2H + 2A) | 0.765 | 1.24 | 1.01 | 0.944 | 1.30 | 0.99 | 1.00 | 1.09 |
| Second ratio | 2H/2A | 0.719 | 0.667 | 0.667 | 0.667 | 0.660 | 0.914 | - | - |
| Third ratio | 2N/2P | - | 1.79 | 3.63 | 5.37 | 1.63 | 3.75 | - | - |
| Fourth ratio | 2N/(P units) | - | 0.207 | 0.210 | 0.207 | 0.126 | 0.218 | - | - |
| Laser processability | | Good | VG | VG | VG | VG | VG | Poor | Fair |

### Reference Signs List

- X1, X2, X3: identification sheet
- M1, M2, M3, M4: distinguishing mark
- 10: backing sheet
- 10a: outer face
- 10b: mount face
- 11: base layer
- 12: writable layer
- 13: background layer
- 14: release layer
- 20: pressure-sensitive adhesive sheet
- 20a: outer face
- 21: substrate layer
- 22: pressure-sensitive adhesive layer
- 22a: adhesive face

## Claims

1. An identification sheet comprising
a pressure-sensitive adhesive sheet including
a pressure-sensitive adhesive layer containing a polyester polyurethane as a principal component.

2. The identification sheet according to claim 1,
wherein the polyester polyurethane is a polyester-polyether polyurethane.

3. The identification sheet according to one of claims 1 and 2,
wherein the polyester polyurethane comprises:
units derived from a multifunctional isocyanate;
units derived from a polycarboxylic acid; and
units derived from a first polyhydric alcohol.

4. The identification sheet according to claim 2,
wherein the polyester-polyether polyurethane comprises:
units derived from a multifunctional isocyanate;
units derived from a polycarboxylic acid;
units derived from a first polyhydric alcohol; and
units derived from a second polyhydric alcohol containing an ether bond.

5. The identification sheet according to claim 4,
wherein, in the polyester-polyether polyurethane, the ratio of the total number of hydroxy groups in the first polyhydric alcohol to the total number of hydroxy groups in the second polyhydric alcohol is 1.5 to 9.0.

6. The identification sheet according to one of claims 4 and 5,
wherein the second polyhydric alcohol to constitute the polyester-polyether polyurethane is a multimer that is a polymer derived from a single type of monomer, and
wherein, in the polyester-polyether polyurethane, the ratio of the total number of hydroxy groups in the first polyhydric alcohol to the total number of units derived from the single type of monomer in the second polyhydric alcohol is 0.1 to 0.4.

7. The identification sheet according to any one of claims 4 to 6,
wherein the second polyhydric alcohol is an aliphatic or alicyclic polyhydric alcohol containing an ether bond.

8. The identification sheet according to claim 7,
wherein the second polyhydric alcohol is a polypropylene glycol.

9. The identification sheet according to any one of claims 3 to 8,
wherein, in the polyester polyurethane, the ratio of the total number of hydroxy groups in the polyhydric alcohol or polyhydric alcohols to the sum of the total number of isocyanate groups in the multifunctional isocyanate and the total number of carboxy groups in the polycarboxylic acid is 0.5 to 1.5.

10. The identification sheet according to any one of claims 3 to 9,
wherein, in the polyester polyurethane, the ratio of the total number of isocyanate groups in the multifunctional isocyanate to the total number of carboxy groups in the polycarboxylic acid is 0.5 to 1.0.

11. The identification sheet according to any one of claims 3 to 10,
wherein the multifunctional isocyanate is an aliphatic or alicyclic multifunctional isocyanate.

12. The identification sheet according to claim 11, wherein the multifunctional isocyanate is at least one selected from the group consisting of:
hexamethylene diisocyanate;
1,3-bis(isocyanatomethyl)cyclohexane;
isophorone diisocyanate;
4,4'-methylenebis(cyclohexyl isocyanate); and
hydrogenated diphenylmethane diisocyanate.

13. The identification sheet according to any one of claims 3 to 12,
wherein the polycarboxylic acid is an aliphatic or alicyclic polycarboxylic acid.

14. The identification sheet according to claim 13,
wherein the polycarboxylic acid is adipic acid.

15. The identification sheet according to any one of claims 3 to 14,
wherein the first polyhydric alcohol is an aliphatic or alicyclic polyhydric alcohol.

16. The identification sheet according to claim 15,
wherein the first polyhydric alcohol is neopentyl glycol.

17. The identification sheet according to claim 4, wherein the multifunctional isocyanate is hexamethylene diisocyanate,
wherein the polycarboxylic acid is adipic acid,
wherein the first polyhydric alcohol is neopentyl glycol, and
wherein the second polyhydric alcohol is a polypropylene glycol.

18. The identification sheet according to any one of claims 1 to 17,
wherein the polyester polyurethane has a softening temperature of 190°C to 280°C.

19. The identification sheet according to any one of claims 1 to 18,
wherein the pressure-sensitive adhesive sheet has a multilayer structure including:
the pressure-sensitive adhesive layer; and
a substrate layer, and
wherein the identification sheet further comprises
a backing sheet having a mount face to which the pressure-sensitive adhesive layer of the pressure-sensitive adhesive sheet is removably attachable.

20. The identification sheet according to claim 19,
wherein the backing sheet has a first outer face bearing a first distinguishing mark, where the first outer face is disposed on opposite side to the mount face, and
wherein the pressure-sensitive adhesive sheet has a second outer face bearing a second distinguishing mark, where the second outer face is disposed in the substrate layer on opposite side to the pressure-sensitive adhesive layer.

21. The identification sheet according to claim 20,
wherein the first distinguishing mark and the second distinguishing mark are each independently selected from an engraved mark and a printed mark.
